# EUROPEAN PATENT APPLICATION

(11) **EP 2 380 565 A1**
(43) Date of publication of application: **26.10.2011**
(21) Application number: 10003632.6
(22) Date of filing: 31.03.2010
(51) Int. Cl.: A61K 31/00, A61K 31/661, A61K 31/685, A61P 31/00

(54) **Compositions for dental treatment comprising Lipoteichoic acids or parts thereof like mono- or polyglycerphosphates**

(71) Applicant: Lunamed AG, 7000 Chur (CH)
(72) Inventor:
(74) Representative: Peters, Hajo

(57) **Abstract**

The present invention refers to compositions for the treatment or prophylaxis of infectious or inflammatory processes in the oral cavity (*cavitas oris*) including dental hygiene, the composition comprising lipoteichoic acids and glycerophosphate derivatives, preferably added into a dental hygiene product like toothpastes, toothgels, creams or rinsing fluids, the use of glycerophosphate derivatives as prophylaxis against bacterial infections of the oral cavity like dental caries, or the use of poly-glycerophosphates for the production of a medicament for the treatment of or prophylaxis against bacterial infections of the oral cavity, like dental caries.

## Description

### Field of the invention

The present invention refers to compositions for the prophylaxis of infectious or inflammatory processes in the oral cavity (*cavitas oris*) including dental hygiene. The composition comprising lipoteichoic acids or parts thereof like mono- or polyglycerophosphates or derivatives, added preferably to dental hygiene products like toothpastes, toothgels, creams or rinsing fluids, can be used as prophylaxis or treatment against gram-positive bacterial infections of the oral cavity and therefore preventing dental caries and gum and soft tissue inflammations.

### Background of the invention

The oral cavity (*cavitas oris*) is one of the first lines of defense of the body which is routinely confronted with microorganisms and viruses many of them potentially causing infections or inflammations. One major focus of this invention thus rests in the prophylaxis against and treatment of bacterial infections and resulting inflammatory reactions and includes especially the field of dental hygiene

Dental hygiene is a very important factor to human health. The public health costs which are invested into curing the effects of lack of dental hygiene are estimated as billions of dollars for the USA or the European Community alone. Most of the damages done to dental structures result from the activity of bacteria investing or adhering to the oral cavity, a majority of them being gram-positive bacteria.

One of the major causes of damage to the dental structure by bacteria is dental caries *(caries dentium),* also known as tooth decay or cavity. Dental caries is a disease where bacterial processes damage hard tooth structure (enamel, dentin and cementum). These tissues progressively break down, producing dental cavities (holes in the teeth). Tooth decay is alleged to be caused by the acid being produced by bacteria which cause damage in the presence of fermentable carbohydrates such as sucrose, fructose, and glucose. Two groups of bacteria are taken as being responsible for initiating caries, Streptococci and Lactobacilli both of them being gram positive. Today, caries remains one of the most common diseases throughout the world. Dental health organizations advocate preventive and prophylactic measures, such as regular oral hygiene and dietary modifications, to avoid dental caries.

Accordingly, it was the objective of the present invention to provide a new form of composition like a dental treatment composition for the prophylaxis and treatment of infectious processes in the oral cavity, including a composition for dental hygiene.

It has now surprisingly been found that administration of lipoteichoic acids and glycerophophate derivatives to the oral cavity results in a highly effective prophylaxis against bacterial infections, thus preventing dental caries.

Thus, the present invention relates to lipoteichoic acids and other glycerophosphate derivatives according to general formula I; 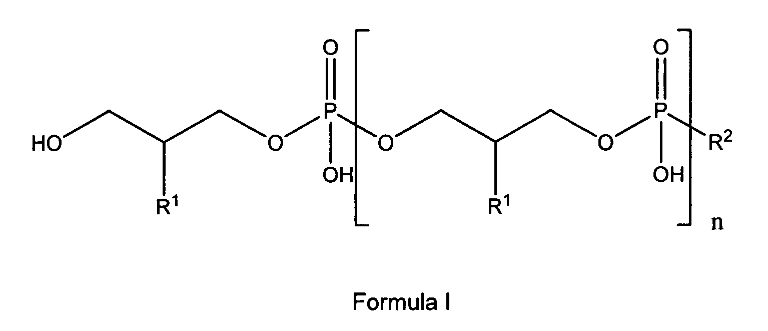 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
either 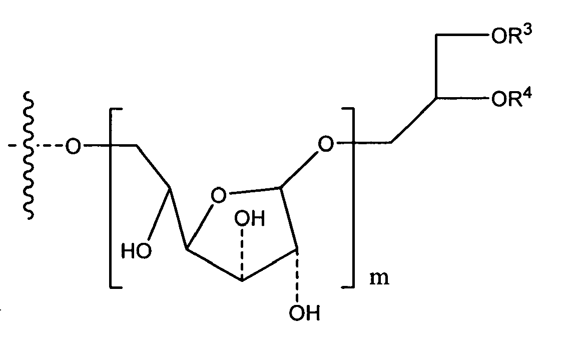 or its tautomeric equivalent 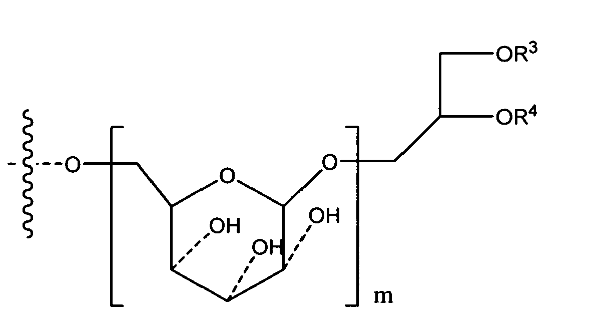 with R³ and R⁴ independently from one another being residues of saturated or unsaturated, unsubstituted fatty acids with 10 to 20 carbon atoms; and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures;
for use in the treatment of or prophylaxis against an inflammatory process caused by an infection, of or against an infection or of or against an infectious process in the *cavitas oris.*

The compounds as described by general formula I and its defined radicals are defined in this invention as "glycerophosphate derivatives of the invention" or as "glycerophosphate derivatives used according to the invention".

*"Cavitas oris"* is the international Latin definition for Oral cavity. According to this application *"cavitas oris"* or oral cavity is defined as the complete area of the mouth including the *vestibulum oris* the area between the frontal part of the teeth and the inner lips as well as the *cavitas oris propria* from the teeth to the pharynx including the hypopharynx the pars laryngia of the pharynx.

"Infection" is defined as the transmission, adherence and penetration of microorganisms into a macroorganism like a plant, an animal or a human. The infecting microorganism might be a virus, a bacterium, a fungus, a worm or a protozoa. In connection with this invention the microorganism preferably is a bacterium, most preferably a gram-positive bacterium. By definition of this application also the transmission and adherence of gram-positive bacteria like streptococci or lactobacilli to the dental structure during or at the beginning of dental caries is defined as an infection.

"infectious process" is defined as the transmission and adherence of microorganisms into or to a macroorganism like a plant, an animal or a human. The infecting microorganism might be a virus, a bacterium, a fungus, a worm or a protozoa. In connection with this invention the microorganism preferably is a bacterium, most preferably a gram-positive bacterium. By definition of this application also the transmission and adherence of gram-positive bacteria like *streptococci* or *lactobacilli* to the dental structure during or at the beginning of dental caries is defined as an infectious process.

"Inflammatory process caused by an infection" is defined as a local inflammatory reaction following the adherence and penetration of a microorganism to or into a macroorganism like a plant, an animal or a human. The infecting microorganism might be a virus, a bacterium, a fungus, a worm or protozoa. In connection with this invention the microorganism preferably is a bacterium, most preferably a gram-positive bacterium.

In one preferred embodiment referring to a glycerol phosphate the glycerophosphate derivative used according to the invention is a compound of formula I being a glycerol phosphate according to any of the following formulas V, Va, Vb or Vc: 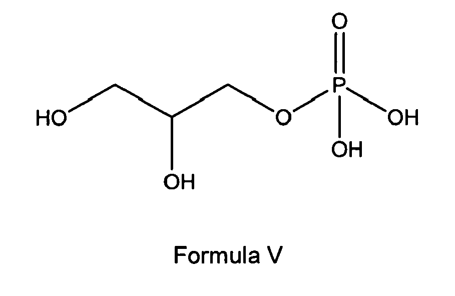 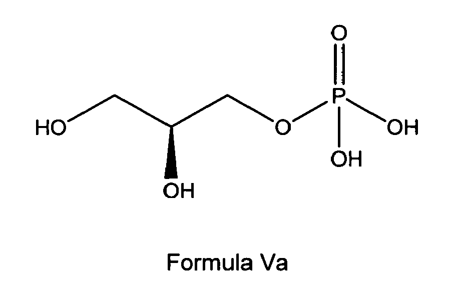 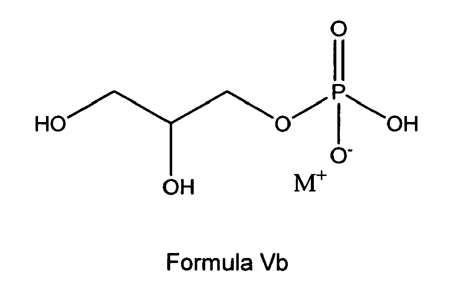 or 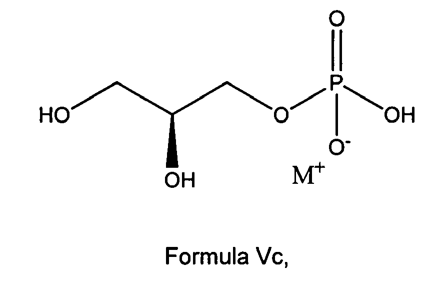 wherein M⁺ is selected from positively charged ions, such as alkali metal ions or positively charged primary, secondary, tertiary or quaternary ammonium ions, or M⁺ is a sodium ion.

The compound/s as described by general formula V, Va, Vb, Vc and their defined radicals above is/are defined in this invention as "glycerol phosphate/s of the invention" or "glycerol phosphate/s used according to the invention".

In the embodiment referring to a glycerol phosphate the compound of formula I is selected from free glycerol phosphate, sodium glycerol phosphate, potassium glycerol phosphate or magnesium glycerol phosphate, preferably is sodium glycerol phosphate (or free glycerol phosphate).

In one preferred embodiment referring to a polyglycerol phosphate the glycerophosphate derivative used according to the invention is a polyglycerol phosphate according to general formula I; 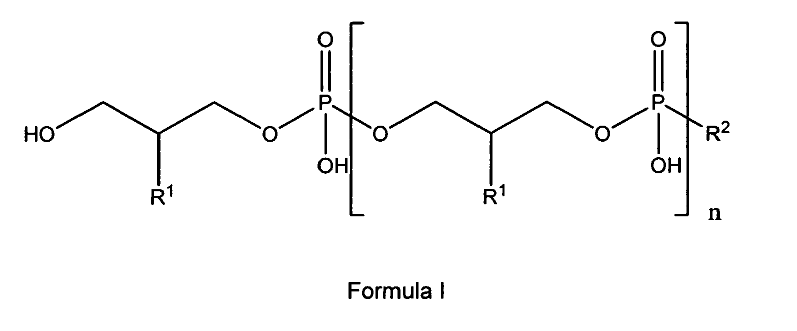 wherein
n is 1 to 11,
R¹ is either H, OH or OMe or OEt and
R² is OH or -O-CH₂-CH(R¹)-CH₂-OH;
with the compounds of formula I being present either in free form or as physiologically acceptable salt; in form of any of their structural isomers including mixtures thereof, preferably in pure enantiomeric or diastereomeric form or any mixture thereof including racemic mixtures. These compounds as well as those described by general formula II, IIa, IIb, IIc, IId or IIe and their defined radicals below are defined in this invention as "polyglycerol phosphate/s of the invention" or "polyglycerol phosphate/s used according to the invention".

In a preferred embodiment referring to a polyglycerol phosphate of the invention the glycerophosphate derivative used according to the invention is a polyglycerol phosphate according to any of general formulas II, IIa, IIb, IIc, IId or IIe 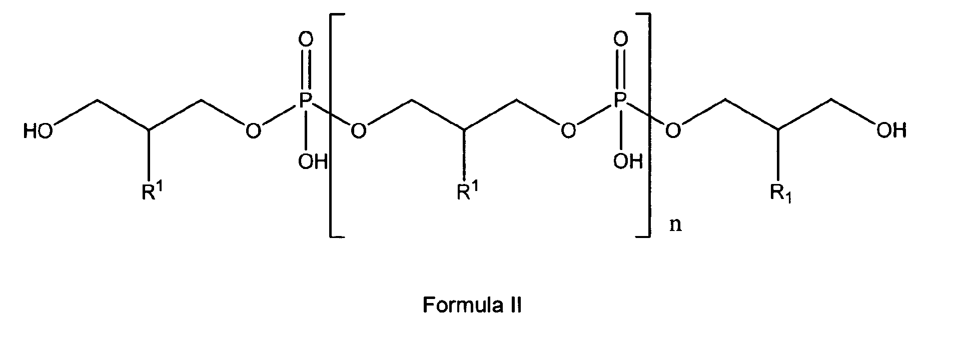 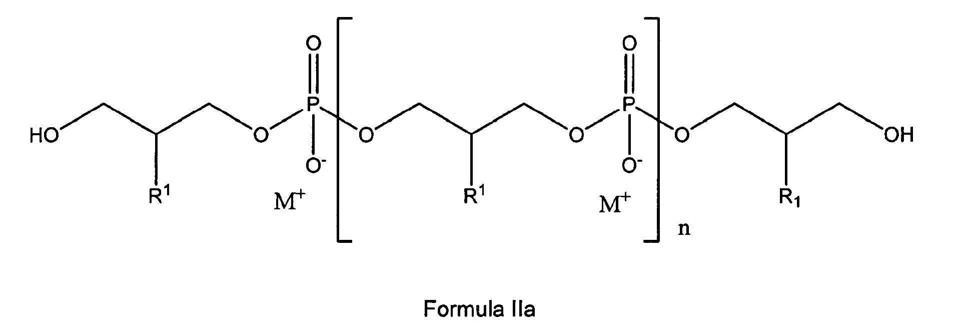 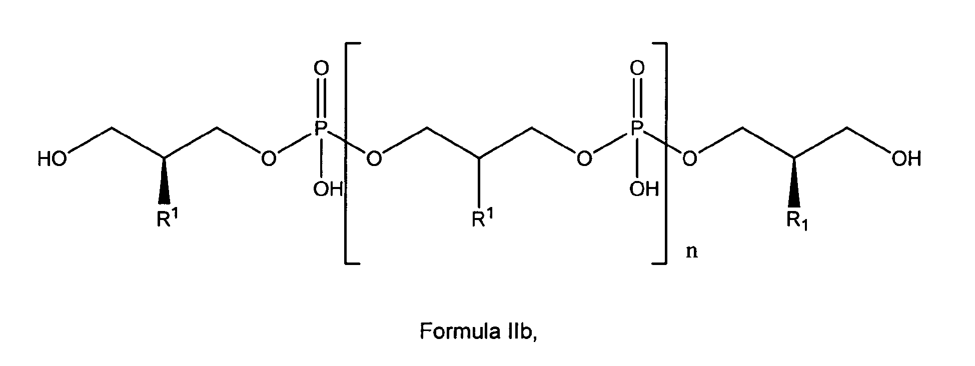 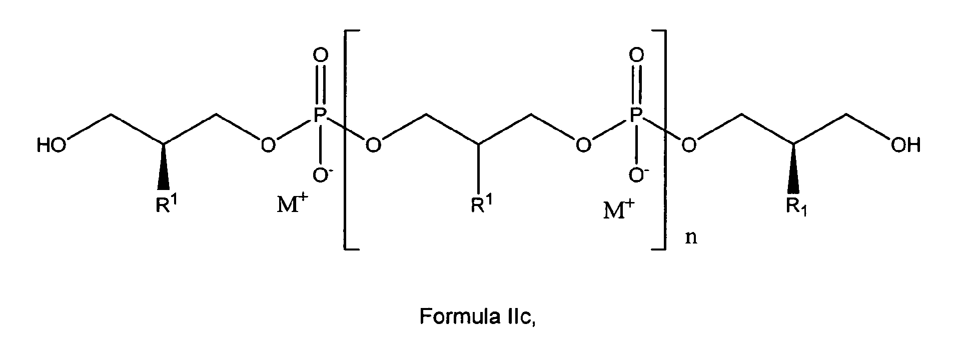 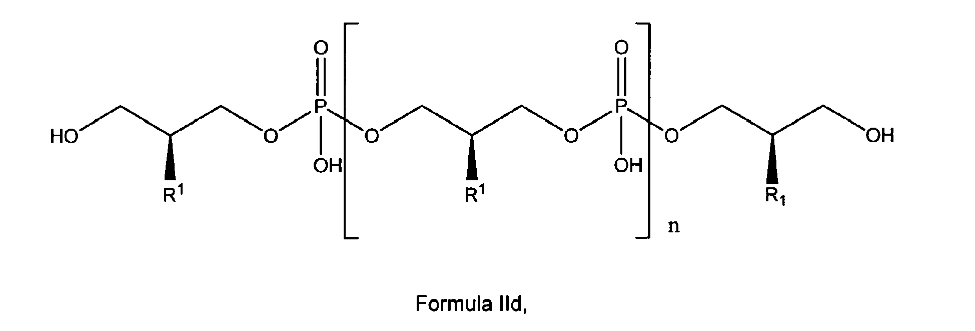 or 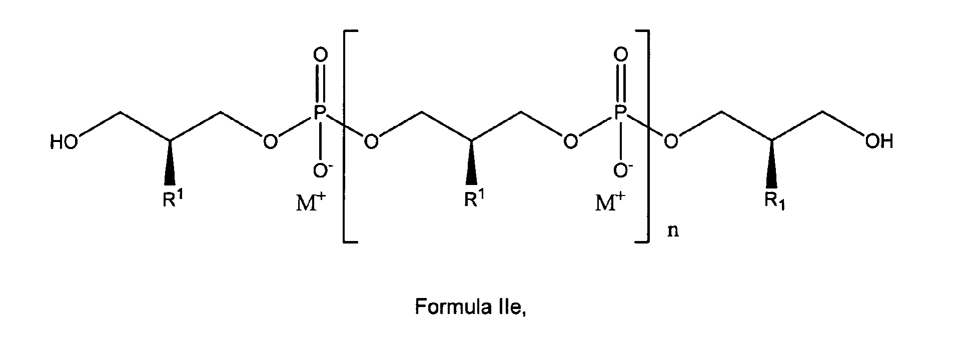 wherein
n is 0 to 10, or n is 1 to 10;
R¹ is either H, OH or OMe or OEt, or R¹ is OH; and
M⁺ is selected from positively charged ions, such as alkali metal ions or positively charged primary, secondary, tertiary or quaternary ammonium ions, or M⁺ is a sodium ion.

In a preferred embodiment referring to a polyglycerol phosphate of the invention the glycerophosphate derivative used according to the invention is a polyglycerol phosphate according to general formula IId 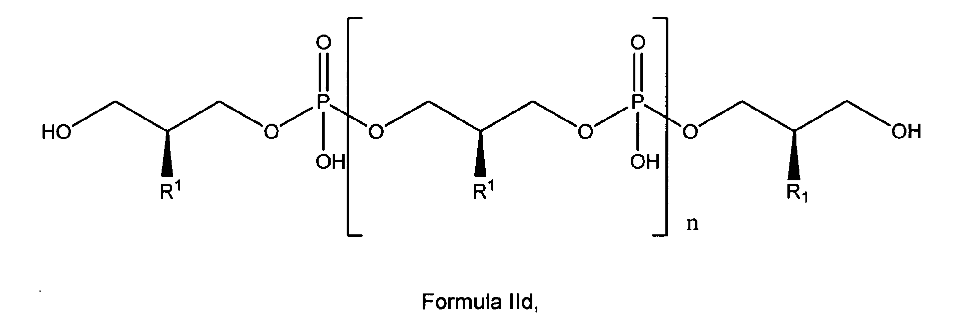 wherein
n is 3; and
R¹ is OH;
either in free form or as physiologically acceptable salt.
This compound is defined as "PGP1" hereinafter.
The compound can be synthesized according to the PhD-thesis of A. Stadelmaier (2003) at the University of Konstanz and the general synthetic principle is also described in Stadelmaier et al, "Synthesis of the First Fully Active Lipoteichoic Acid", Angew. Chem. International Edition (2003); 42 (8), p. 916-920.

One aspect and embodiment of the invention refers to a teichoic acid for use in the treatment of or prophylaxis against an inflammatory process caused by an infection, of or against an infection or of or against an infectious process in the *cavitas oris.* "Teichoic acids" as used herein is an inclusive term including the a) lipoteichoic acids (LTA) and b) the wall teichoic acids (WTA) form a major part of the cell wall of gram-positive bacteria.

A very preferred aspect and embodiment of the invention refers to a (general) lipoteichoic acid for use in the treatment of or prophylaxis against an inflammatory process caused by an infection, of or against an infection or of or against an infectious process in the *cavitas oris.* There are many lipoteichoic acids known which form a major part of the cell wall of gram-positive bacteria.

The known lipoteichoic acids include synthetic, semisynthetic and naturally occurring lipoteichioic acids. A very broad introduction on the synthesis of as well as on synthetic lipoteichoic acids can be found in Pedersen, Christian Marcus; Schmidt, Richard R. (Edited by Moran, Anthony P), Microbial Glycobiology (2009), 455-476 with all lipoteichoic acids described therein herewith included by reference n this application as examples of the lipoteichoic acids to be used.. A further overview of the lipoteichoic acids - divided in Types I to IV - can be found in Greenberg et al., Infect Immun. 1996 Aug;64(8):3318-25. All II lipoteichoic acids Type I, Type II, Type III and Type IV as well as all those described therein (like e.g. in tables 1 or 2) herewith included by reference in this application as examples of the lipoteichoic acids to be used.

The (general) lipoteichoic acids as defined in this application are comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid.

Lipoteichoic acids falling under this definition according to this invention are called "(general) lipoteichoic acids" already before and hereinafter.

The glycolipid acts as an anchor in the cell membrane. The glycolipids are by definition of the invention lipids of the membrane in which one or more mono- or oligosaccharids are bound via a glycosidic bond to a lipid molecule. The lipid molecule is consisting of fatty acids being bound by an ester bond to a glycerol or by an amid bond to a sphingosine.

Preferably the glycolipid is a glyceroglycolipid in which one or more mono- or oligosaccharids are bound via a glycosidic bond to a lipid molecule consisting of fatty acids being bound by an ester bond to the glycerol. Preferably the glyceroglycolipid comprises a diacylglycerol with two fatty acid chains of 10 to 20 carbon atoms each being bound by an ester bond to the glycerol. Preferably the fatty acid chains are linear or branched and non-substituted and/or have a length of 10 to 20 carbon atoms each, most preferably 12, 14, 16 or 18 carbon atoms.

Most (general) lipoteichoic acids would be covered by the general formula I and its substituents as described above and directly below and some preferred embodiments are described below, but some are unusual. These unusual include the lipoteichoic acid from *Streptococcus pneumoniae* (see below) whose synthesis was recently described by Pedersen et al., in Angew. Chem. (2010), 122, 1 - 7. 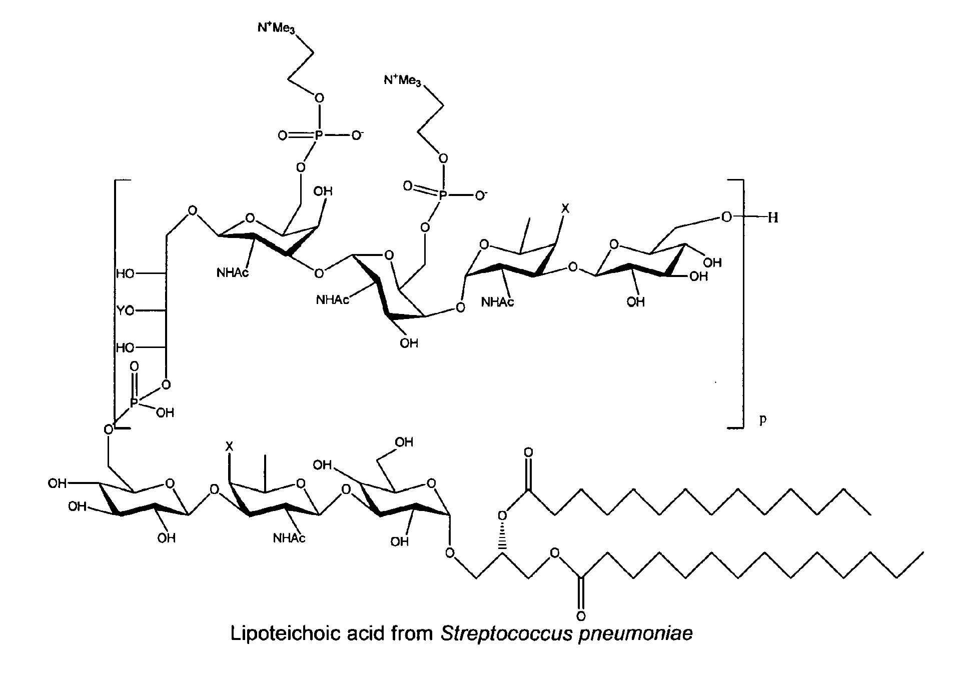 wherein
in one case X is NH₃⁺, or NHAc; Y is H, D-Ala, or α-GalNAc; and p is up to 8;
in the other case X is NH₃⁺; Y is H; and p is 1.

In one preferred embodiment of the invention (referring to a lipoteichoic acid) the glycerophosphate derivative used according to the invention is a lipoteichoic acid according to general formula I 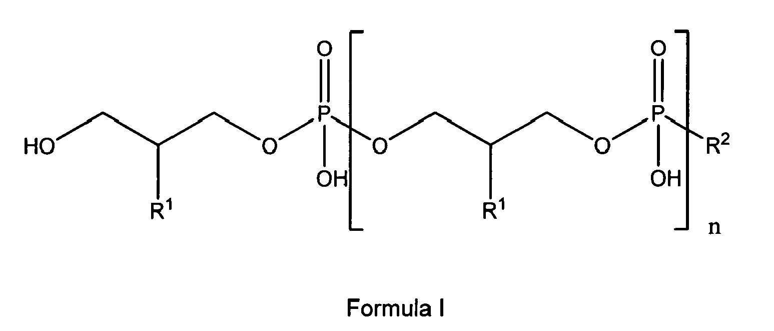 wherein
the mean value of n is 8 to 40,
R¹ is either OH or O-D-alanyl and
R² is either 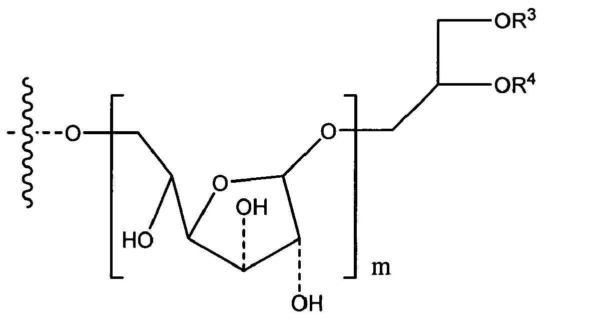 or its tautomeric equivalent 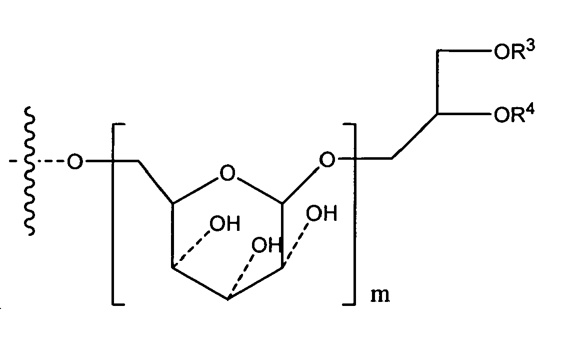 with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

In one further even more preferred embodiment of the invention (referring to a lipoteichoic acid) the glycerophosphate derivative used according to the invention is a lipoteichoic acid according to general formula I 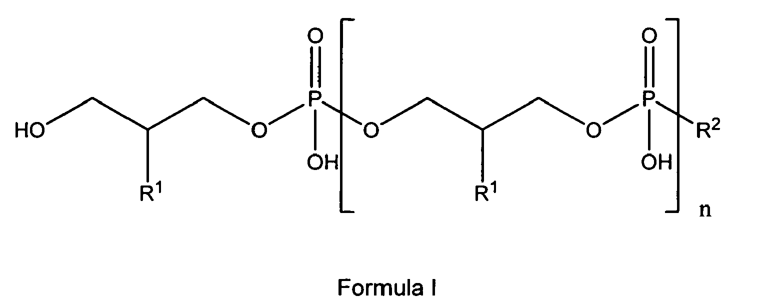 wherein
the mean value of n is 9,
R¹ is either OH or O-D-Alanyl and
R² is 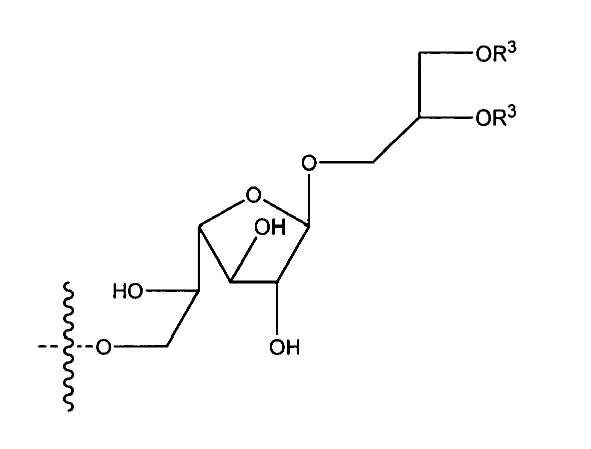 with R³ being residues of saturated or unsaturated fatty acids with 12, 14, 16 or 18 carbon atoms,
   with the compounds of formula I being present either in free form or as physiologically acceptable salt; in form of any of their structural isomers including mixtures thereof, preferably in pure enantiomeric or diastereomeric form or any mixture thereof including racemic mixtures. These compounds (lipoteicoic acids) defined directly above as well as those described by general formula III, IIIa, IIIb, or IIIc and their defined radicals below (as well as any lipoteichoic acid to be isolated from the Streptococcus sp. PT strain DSM 8747) are defined in this invention as "lipoteichoic acid of the invention" or "lipoteichoic acid used according to the invention".
   In a further embodiment the invention relates to a purified lipoteichoic acid isolatable from the Streptococcus sp. PT strain DSM 8747, preferably containing a beta-galactofuranosyl(1-3)glycerol-di-ester moiety, for use in the treatment of or prophylaxis against an inflammatory process caused by an infection, of or against an infection or of or against an infectious process in the *cavitas oris.*

In one preferred embodiment (referring to a lipoteichoic acid) the lipoteichoic acid used according to the invention is a compound according to any of general formulas III, IIIa, IIIb, or IIIc 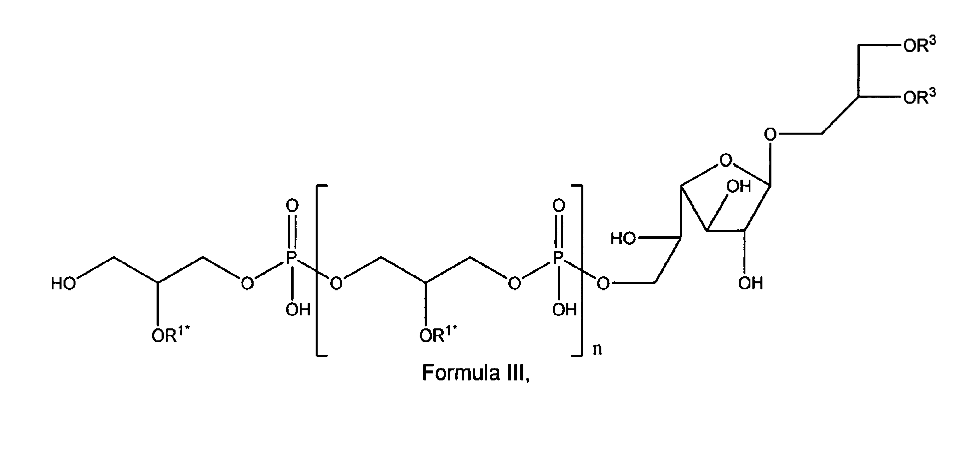 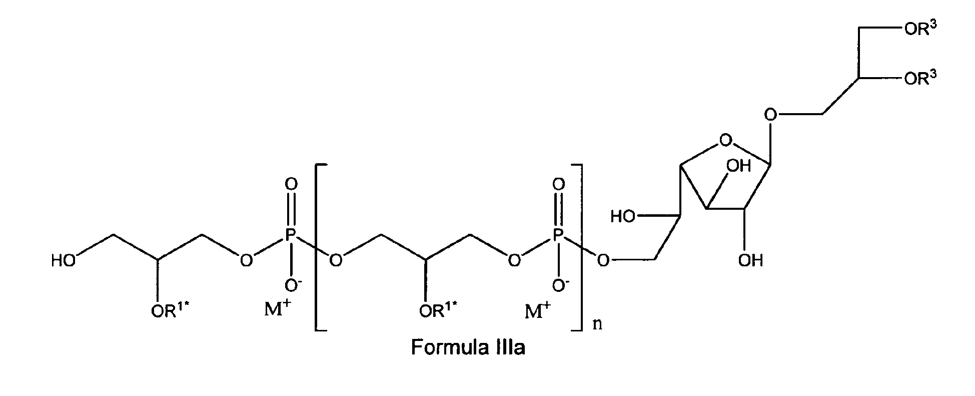 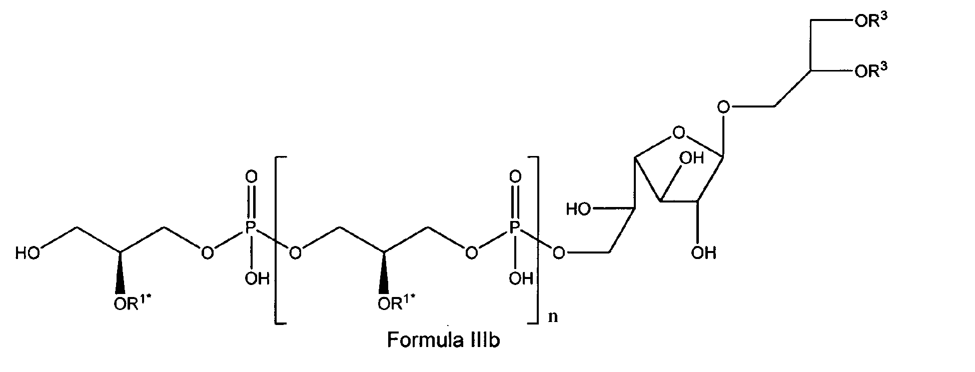 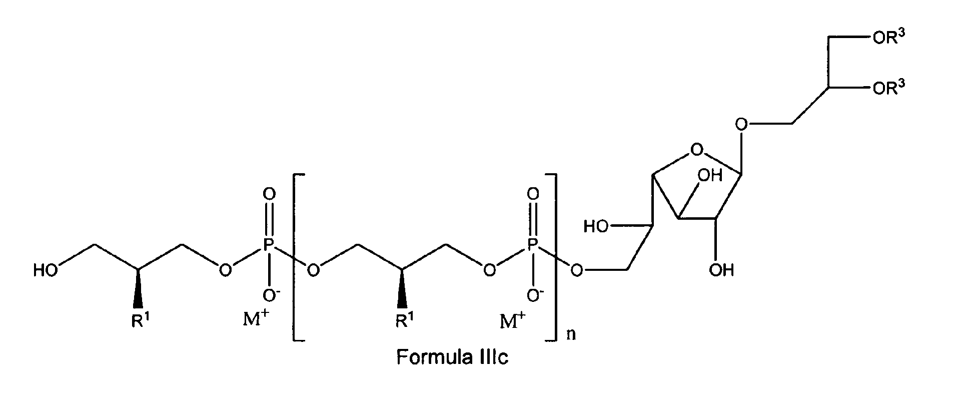 with
n having a mean value of 9, preferably being 6 to 12 while having a mean value of 9,
R¹* being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

In one preferred embodiment (referring to a lipoteichoic acid) the lipoteichoic acid used according to the invention is a purified lipoteichoic acid (hereinafter called LTA-T) isolated from bacteria of the genus *streptococcus,* preferably from *streptococcus sp.,* most preferably from *streptococcus sp.* (DSM 8747).

Lipoteichoic acids - especially the lipoteichoic acid (LTA-T) - are described in the PCT-application WO 96/23896 together with its antitumor cholesterol-lowering activity and ways of isolating the LTA-T. The whole disclosure of WO06/23896 is included in this application by way of reference.

In one further embodiment (referring to a lipoteichoic acid) the lipoteichoic acid used according to the invention being a compound according to general formula I is a lipoteichoic acid of *staphylococcus aureus* (below): 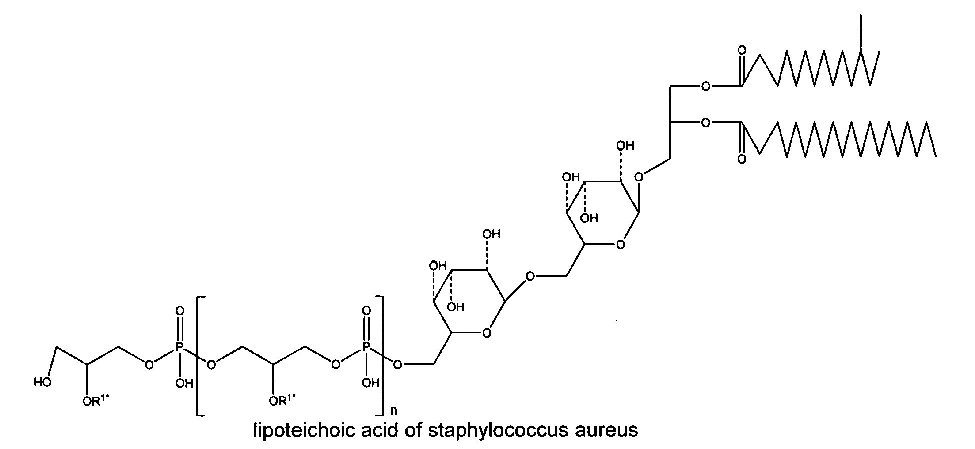 , wherein X is H or D-ala and the mean value of "n" is 16 to 40.

In a preferred embodiment referring to a glycerophosphate derivative, a glycerol phosphate, a polyglycerol phosphate or a lipoteichoic acid (and also a (general) lipoteichoic acid) used according to the invention the infection is a bacterial infection, the infectious process is bacterial infectious process and/or the inflammation is caused by a bacterial infection, preferably wherein the infection is caused by gram-positive bacteria, the infectious process is caused by gram-positive bacteria and/or the inflammation is caused by gram-positive bacteria.

Preferably the gram-positive bacteria are lactobacilli and streptococci.

In a preferred embodiment referring to a glycerophosphate derivative, a glycerol phosphate, a polyglycerol phosphate or a lipoteichoic acid (and also a (general) lipoteichoic acid) used according to the invention the infectious process is *caries dentium* or periodontal diseases preferably is *caries dentium* or the use is in the treatment of or prophylaxis against *caries dentium* or periodontal diseases preferably of or against *caries dentium.*

"Dental caries = *caries dentium",* is well-known and also known as tooth decay or cavity being a disease where bacterial processes damage hard tooth structure (enamel, dentin and cementum).

"Periodontal diseases" is a general description of all diseases of the peridontium. This includes especially bacterially caused marginal periodontal diseases.

In a preferred embodiment referring to a glycerophosphate derivative, a glycerol phosphate, a polyglycerol phosphate or a lipoteichoic acid (also a (general) lipoteichoic acid) used according to the invention the use includes use of an oral or dental hygiene product, preferably a use wherein the glycerophosphate derivative is used in form of a toothpaste, a toothgel, a cream or a rinsing fluid.

"Oral or dental hygiene product" is defined in this invention as any hygiene product used in the oral cavity and especially on the teeth for a hygienic effect. Examples include a toothpaste, a toothgel, a cream or a rinsing fluid (mouth wash). All of these products are either used for treating a already occurred disorder like bad breath, dental caries, (marginal) periodontal diseases, inflammation etc. or - most often as prophylaxis - to prevent such an event (like establishing dental caries) from happening.

In a preferred embodiment referring to a glycerophosphate derivative, a glycerol phosphate, a polyglycerol phosphate or a lipoteichoic acid (also a (general) lipoteichoic acid) used according to the invention the use includes use of a composition - like (or included in) an oral or dental hygiene product - wherein the glycerophosphate derivative according to general formula I or glycerol phosphate, polyglycerol phosphate or lipoteichoic acid is present in an amount between 0.1 to 100 mg/ml, preferably 1 to 10 mg/ml, more preferably 2 to 5 mg/ml, most preferably 3 to 3.5 mg/ml or 0.02 to 2.0 weight-%, preferably 0.05 to 1.0 mg/ml, more preferably 0.1 to 0.75 weight-%, most preferably 0.2 to 0.4 weight-% of the composition in the oral or dental hygiene product.

In another preferred embodiment referring to a glycerophosphate derivative, a glycerol phosphate, a polyglycerol phosphate or a lipoteichoic acid (also a (general) lipoteichoic acid) used according to the invention the use includes use of a composition - like an oral or dental hygiene product - wherein the glycerophosphate derivative according to general formula I or glycerol phosphate, polyglycerol phosphate or lipoteichoic acid is present in an amount between 0.1 to 100 mg/ml, preferably 0.5 to 10 mg/ml, more preferably 0.7 to 7 mg/ml, most preferably 2 to 5 mg/ml or most preferably 3 to 3.5 mg/ml (especially if a lipoteichoic acid) of the composition in the oral or dental hygiene product.

In a preferred embodiment referring to a glycerophosphate derivative, a glycerol phosphate, a polyglycerol phosphate or a lipoteichoic acid (also a (general) lipoteichoic acid) used according to the invention wherein the use includes use of a composition - like (or included in) an oral or dental hygiene product - wherein the composition in the oral or dental hygiene product further comprises substances selected from
a fluoride or a sweetener or a preservative,
preferably (in case of a tooth paste, tooth gel or tooth cream) selected from a fluoride, an abrasive, an enzyme, a surfactant, an oxidizer, a flavouring substance, a sweetener, a binder, a humectant, or a preservative, or
preferably (in case of a rinsing fluid) selected from water, an alcohol, a buffering substance, a sweetener, a flavouring substance, a preservative, a fluoride, an enzyme, or an oxidizer.

In a preferred embodiment referring to a glycerophosphate derivative, a glycerol phosphate, a polyglycerol phosphate or a lipoteichoic acid (also a (general) lipoteichoic acid) used according to the invention wherein the use includes use of a composition - like (or included in) an oral or dental hygiene product - wherein the composition in the oral or dental hygiene product further shows a pH ≥ 5.5, preferably a pH ≥ 7.0.

The "abrasive" is selected e.g. from calcium carbonate or silica like powdered white mica or calcium sodium phosphosilicate or hydrated silica.

The "surfactant" is selected e.g. from sodium lauryl sulfate (SLS) or cocamidopropyl betaine.

The "oxidizer" is selected from hydrogen peroxide.

The "alcohol" preferably is ethanol.

The "enzyme" is selected from biological detergents.

The "fluoride" is selected from e.g. sodium fluoride, olaflur, dectaflur, hexafluorosilicic acid (H₂SiF₆) and its sodium salt (Na₂SiF₆), preferably is sodium fluoride.

The "binder" is selected from e.g. hydroxyethyl cellulose, polyethylene glycol, or gums like xanthan gum or cellulose gum or carrageenan.

The "humectant" is selected from e.g. propylene glycol or glycerine or sorbitol

The "sweetener" includes artificial sweeteners such as sorbitol, sucralose, xylitol or saccharin.

The "buffer" is selected from e.g. phosphate buffered saline (PBS).

The "preservative" is selected from e.g. sodium benzoate or parabenes like methylparaben or propylparaben.

Other substances that might be comprised in the composition include: colorants like titanium dioxide, baking soda, tetrasodium pyrophosphate, triclosan, sodium hydroxide, vitamins, herbs, aromas like limonene or herbal oils, film builders like Acrylates/ C10-30 Alkyl Acrylate crosspolymer or zinc salts.

In a further aspect the invention relates to a dental treatment composition comprising a glycerophosphate derivative according to general formula I or glycerol phosphate/s according to the invention, polyglycerol phosphate according to the invention or lipoteichoic acid according to the invention as described and defined above (also a (general) lipoteichoic acid).

Accordingly, this aspect of the invention relates to a dental treatment composition comprising a glycerophosphate derivative according to general formula I; 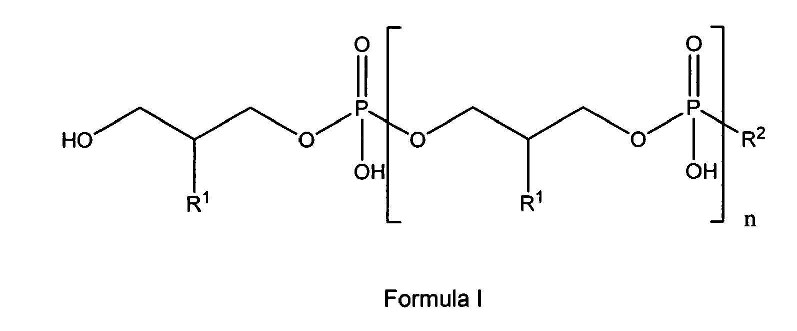 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
either 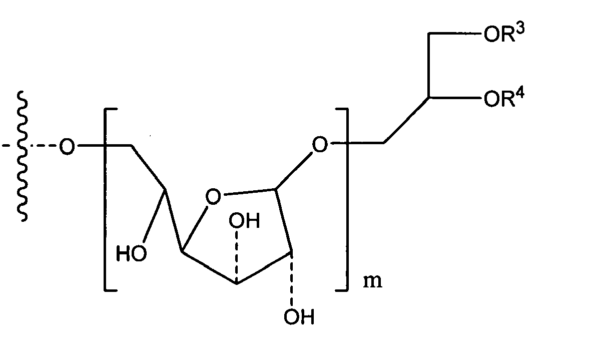 or its tautomeric equivalent 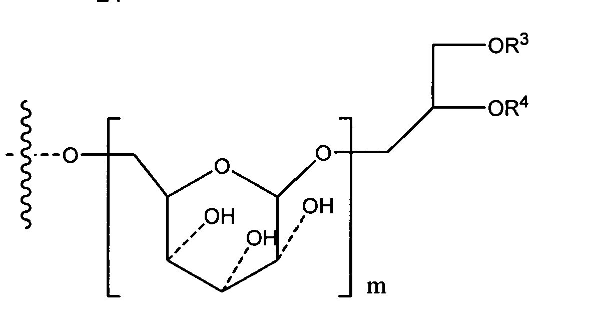 with R³ and R⁴ independently from one another being residues of saturated or unsaturated, unsubstituted fatty acids with 10 to 20 carbon atoms; and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

"Dental treatment composition" is defined in this invention as a composition of matter to be used in the treatment of the teeth. Examples in which this composition may be included include a toothpaste, a toothgel, a cream or a rinsing fluid (mouth wash). The dental treatment composition may either be used for treating a already occurred disorder like established dental caries or periodontal diseases or - most often - as prophylaxis to prevent such an event (like establishing dental caries) from happening.

In a further embodiment of the dental treatment composition according to the invention the composition is for use in the treatment of or prophylaxis against an infection or an infectious process, preferably the composition is for the treatment of or prophylaxis against a bacterial infection and/or bacterial infectious process, more preferably wherein the infection is caused by gram-positive bacteria, and/or the infectious process is caused by gram-positive bacteria. Preferably the infection or infectious process is *caries dentium* or periodontal diseases preferably is *caries dentium.*

In a further embodiment of the dental treatment composition according to the invention the composition is in form of an oral or dental hygiene product, preferably in form of a toothpaste, a toothgel, a cream or a rinsing fluid.

In a further embodiment of the dental treatment composition according to the invention the glycerophosphate derivative according to general formula I or glycerol phosphate, polyglycerol phosphate or lipoteichoic acid (also (general) lipoteichoic acid) as described and defined above is present in an amount between 0.1 to 100 mg/ml, preferably 1 to 10 mg/ml, more preferably 2 to 5 mg/ml, most preferably 3 to 3.5 mg/ml or 0.02 to 2.0 weight-%, preferably 0.05 to 1.0 mg/ml, more preferably 0.1 to 0.75 weight-%, most preferably 0.2 to 0.4 weight-% of the composition.

In a further embodiment of the dental treatment composition according to the invention the glycerophosphate derivative according to general formula I or glycerol phosphate, polyglycerol phosphate or lipoteichoic acid (also (general) lipoteichoic acid) as described and defined above is present in an amount between 0.1 to 100 mg/ml, preferably 0.5 to 10 mg/ml, more preferably 0.7 to 7 mg/ml, most preferably 2 to 5 mg/ml or most preferably 3 to 3.5 mg/ml (especially if a lipoteichoic acid) of the composition in the product.

In a further embodiment of the dental treatment composition according to the invention the dental treatment composition further comprises auxiliary substances selected from
a fluoride or a sweetener,
   preferably (in case of a tooth paste, tooth gel or tooth cream) selected from a fluoride, an abrasive, an enzyme, a surfactant, an oxidizer, a flavouring substance, a sweetener, a binder, a humectant or a preservative, or
   preferably (in case of a rinsing fluid) selected from water, an alcohol, a buffering substance, a sweetener, a flavouring substance, a preservative, a fluoride, an enzyme, or an oxidizer.

The dental treatment composition preferably shows a pH ≥ 5.5, preferably a pH ≥ 7.0.

In a further aspect the invention relates to a dental hygiene product comprising a glycerophosphate derivative according to general formula I or glycerol phosphate/s according to the invention, polyglycerol phosphate according to the invention or lipoteichoic acid according to the invention (also a (general) lipoteichoic acid) as described and defined above. Preferably the dental hygiene product further comprises a fluoride, most preferably selected from sodium fluoride, olaflur, dectaflur or hexafluorosilicic acid (H₂SiF₆) and its sodium salt (Na₂SiF₆), especially sodium fluoride.

Accordingly, this aspect of the invention relates to a dental hygiene product comprising a glycerophosphate derivative according to general formula I; 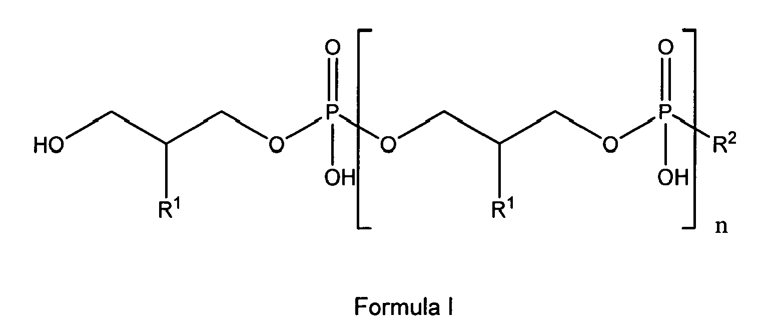 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
   either 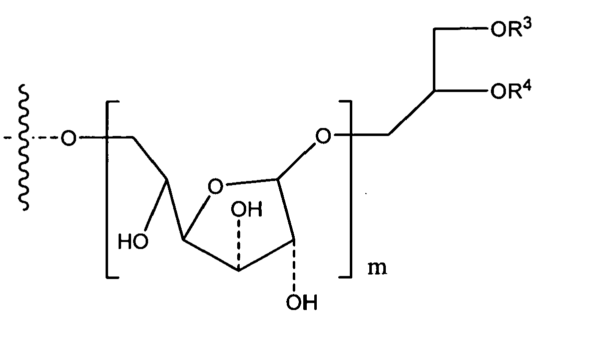 or its tautomeric equivalent 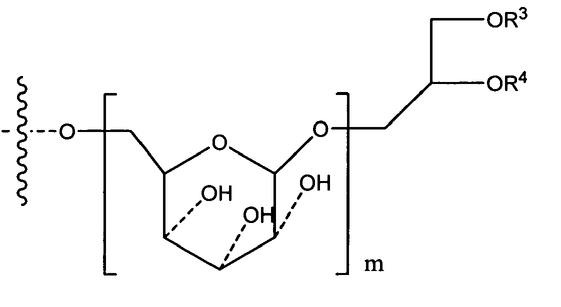 with R³ and R⁴ independently from one another being residues of saturated or unsaturated, unsubstituted fatty acids with 10 to 20 carbon atoms; and m being selected from 1 to 3,
   with the compounds of formula I being present either in free form or as physiologically acceptable salt; in form of any of their structural isomers including mixtures thereof, preferably in pure enantiomeric or diastereomeric form or any mixture thereof including racemic mixtures.

In a further aspect the invention relates to the use of a glycerophosphate derivative according to general formula I or glycerol phosphate/s according to the invention, polyglycerol phosphate according to the invention or lipoteichoic acid according to the invention (also a (general) lipoteichoic acid) as described and defined above in the production of a dental hygiene product, especially a dental hygiene product for the prophylaxis against dental caries or periodontal diseases preferably against *caries dentium.* Accordingly, this aspect of the invention relates to the use of a glycerophosphate derivative according to general formula I; 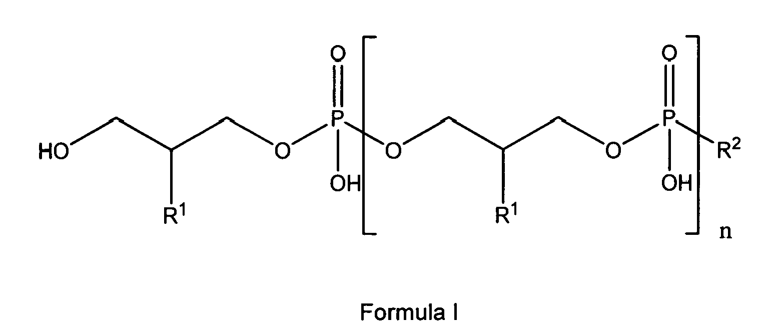 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
   either 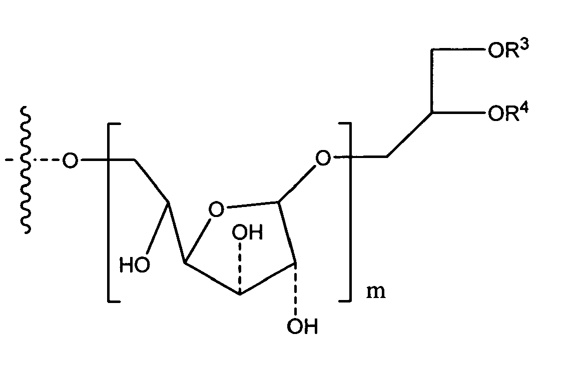 or its tautomeric equivalent 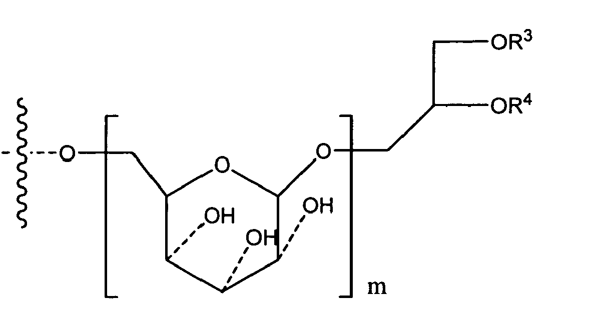 with R³ and R⁴ independently from one another being residues of saturated or unsaturated, unsubstituted fatty acids with 10 to 20 carbon atoms; and m being selected from 1 to 3,
   with the compounds of formula I being present either in free form or as physiologically acceptable salt; in form of any of their structural isomers including mixtures thereof, preferably in pure enantiomeric or diastereomeric form or any mixture thereof including racemic mixtures;
in the production of a dental hygiene product, especially a dental hygiene product for the prophylaxis against dental caries or periodontal diseases preferably against *caries dentium.*

As already defined above a "dental hygiene product" is defined in this invention as any hygiene product used in the oral cavity and especially on the teeth for a hygienic effect. Examples include a toothpaste, a toothgel, a cream or a rinsing fluid (mouth wash). All of these products are either used for treating a already occurred disorder like bad breath, dental caries, (marginal) periodontal diseases, inflammation etc. or - most often as prophylaxis - to prevent such an event (like establishing dental caries) from happening.

Preferably the dental hygiene product is used for the treatment of or prophylaxis against an infection (e.g a bacterial infection preferably by gram-positive bacteria), an infectious process (e.g. a bacterial infectious process preferably by gram-positive bacteria) and/or an inflammation caused by an infection (e.g a bacterial infection preferably by gram-positive bacteria). Most preferably this use is the treatment of or prophylaxis against *caries dentium* or periodontal diseases preferably of or against *caries dentium.*

The dental hygiene product could also be used to prevent adherence - e.g. through competitive inhibition - of bacteria to dental surfaces. This may be useful for the treatment of or prophylaxis against an infection (e.g a bacterial infection preferably by gram-positive bacteria), an infectious process (e.g. a bacterial infectious process preferably by gram-positive bacteria) and/or an inflammation caused by an infection (e.g a bacterial infection preferably by gram-positive bacteria). Most preferably this may be useful in the treatment of or prophylaxis against *caries dentium* or periodontal diseases preferably of or against *caries dentium.*

The dental hygiene product preferably takes the form of a toothpaste, a toothgel, a cream or a rinsing fluid and/or the glycerophosphate derivative according to general formula I or glycerol phosphate, polyglycerol phosphate or lipoteichoic acid of the invention (also (general) lipoteichoic acid) is present in an amount between 0.1 to 100 mg/ml, preferably 1 to 10 mg/ml, more preferably 2 to 5 mg/ml, most preferably 3 to 3.5 mg/ml or 0.02 to 2.0 weight-%, preferably 0.05 to 1.0 mg/ml, more preferably 0.1 to 0.75 weight-%, most preferably 0.2 to 0.4 weight-% of the product. It may also be present in an amount between 0.1 to 100 mg/ml, preferably 0.5 to 10 mg/ml, more preferably 0.7 to 7 mg/ml, most preferably 2 to 5 mg/ml or most preferably 3 to 3.5 mg/ml (especially if a lipoteichoic acid) of the composition in the product. Further the product may further comprise substances selected from:
a fluoride or a sweetener,
preferably (in case of a tooth paste, tooth gel or tooth cream) selected from a fluoride, an abrasive, an enzyme, a surfactant, an oxidizer, a flavouring substance, a sweetener, a binder, a humectant or a preservative, or
preferably (in case of a rinsing fluid) selected from water, an alcohol, a buffering substance, a sweetener, a flavouring substance, a preservative, a fluoride, an enzyme, or an oxidizer.

The dental hygiene product preferably shows a pH ≥ 5.5, preferably a pH ≥ 7.0.

In a further aspect the invention relates to a use of a glycerophosphate derivative according to general formula I or glycerol phosphate/s according to the invention, polyglycerol phosphate according to the invention or lipoteichoic acid according to the invention (also a (general) lipoteichoic acid) as described and defined above in the production of a pharmaceutical composition for the treatment of or prophylaxis against an inflammatory process caused by an infection, of or against an infection or of or against an infectious process in the *cavitas oris.*

Accordingly, this aspect of the invention relates to the use of a glycerophosphate derivative according to general formula I; 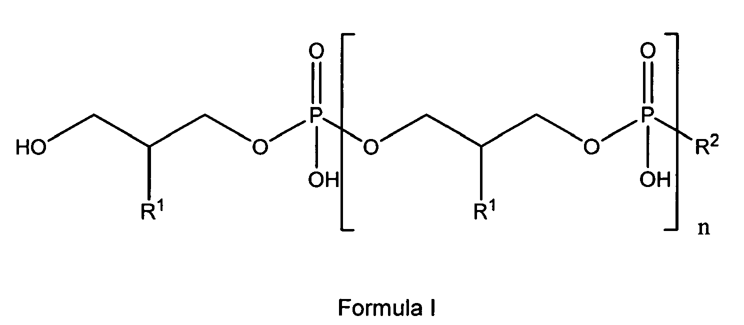 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
   either 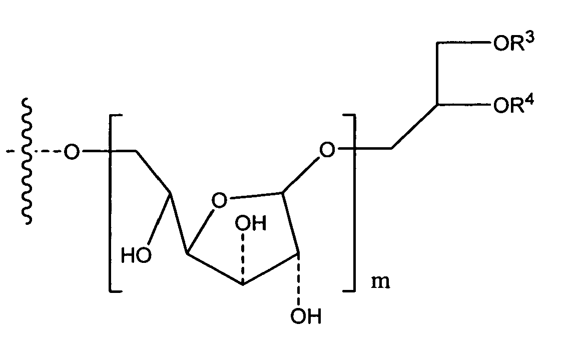 or its tautomeric equivalent 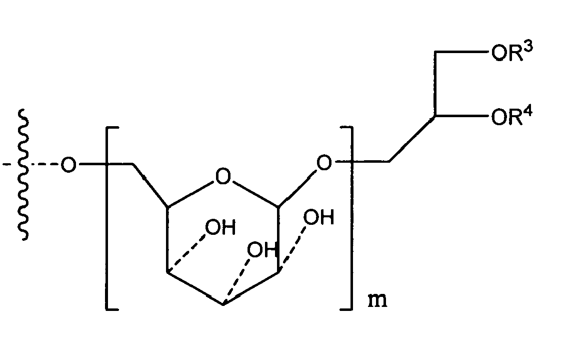 with R³ and R⁴ independently from one another being residues of saturated or unsaturated, unsubstituted fatty acids with 10 to 20 carbon atoms; and m being selected from 1 to 3,
   with the compounds of formula I being present either in free form or as physiologically acceptable salt; in form of any of their structural isomers including mixtures thereof, preferably in pure enantiomeric or diastereomeric form or any mixture thereof including racemic mixtures;
   in the production of a pharmaceutical composition for the treatment of or prophylaxis against an inflammatory process caused by an infection, of or against an infection or of or against an infectious process in the *cavitas oris.*

In a further aspect the invention relates to a composition comprising a glycerophosphate derivative according to general formula I or glycerol phosphate/s according to the invention, polyglycerol phosphate according to the invention or lipoteichoic acid according to the invention as described and defined above (also a (general) lipoteichoic acid) and a fluoride. Preferably the fluoride is selected from sodium fluoride, olaflur, dectaflur or hexafluorosilicic acid (H₂SiF₆) and its sodium salt (Na₂SiF₆), most preferably is sodium fluoride.

Accordingly, this aspect of the invention relates to a composition comprising a glycerophosphate derivative according to general formula I; 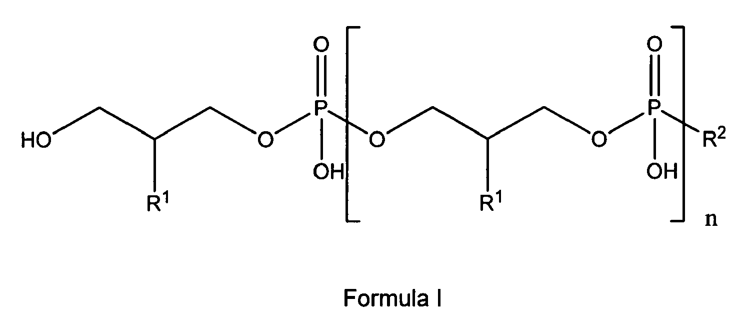 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
   either 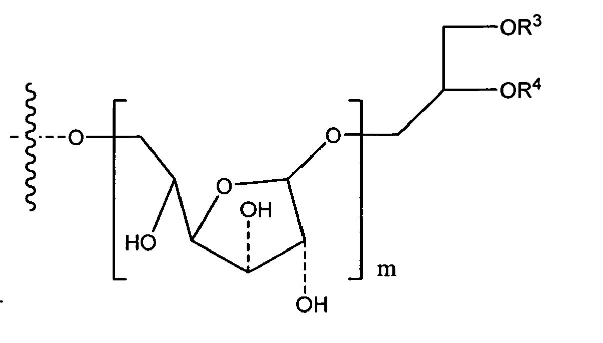 or its tautomeric equivalent 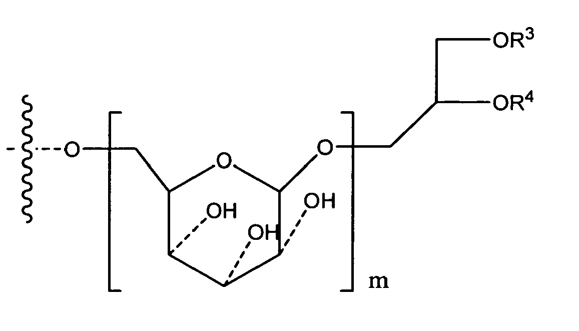 with R³ and R⁴ independently from one another being residues of saturated or unsaturated, unsubstituted fatty acids with 10 to 20 carbon atoms; and m being selected from 1 to 3,
   with the compounds of formula I being present either in free form or as physiologically acceptable salt; in form of any of their structural isomers including mixtures thereof, preferably in pure enantiomeric or diastereomeric form or any mixture thereof including racemic mixtures;
   and a fluoride.

Preferably the composition is used for the treatment of or prophylaxis against an infection (e.g a bacterial infection preferably by gram-positive bacteria), an infectious process (e.g. a bacterial infectious process preferably by gram-positive bacteria) and/or an inflammation caused by an infection (e.g a bacterial infection preferably by gram-positive bacteria). Most preferably this use is the treatment of or prophylaxis against *caries dentium* or periodontal diseases preferably of or against *caries dentium.*

The composition preferably takes the form of a toothpaste, a toothgel, a cream or a rinsing fluid and/or the glycerophosphate derivative according to general formula I or glycerol phosphate, polyglycerol phosphate or lipoteichoic acid of the invention (also a (general) lipoteichoic acid) is present in an amount between 0.1 to 100 mg/ml, preferably 1 to 10 mg/ml, more preferably 2 to 5 mg/ml, most preferably 3 to 3.5 mg/ml or 0.02 to 2.0 weight-%, preferably 0.05 to 1.0 mg/ml, more preferably 0.1 to 0.75 weight-%, most preferably 0.2 to 0.4 weight-% of the product. It may also be present in an amount between 0.1 to 100 mg/ml, preferably 0.5 to 10 mg/ml, more preferably 0.7 to 7 mg/ml, most preferably 2 to 5 mg/ml or most preferably 3 to 3.5 mg/ml (especially if a lipoteichoic acid) of the composition in the product. Further the composition may further comprise substances selected from:
a fluoride or a sweetener,
preferably (in case of a tooth paste, tooth gel or tooth cream) selected from a fluoride, an abrasive, an enzyme, a surfactant, an oxidizer, a flavouring substance, a sweetener, a binder, a humectant or a preservative, or
preferably (in case of a rinsing fluid) selected from water, an alcohol, a buffering substance, a sweetener, a flavouring substance, a preservative, a fluoride, an enzyme, or an oxidizer.

The composition preferably also shows a pH ≥ 5.5, preferably a pH ≥ 7.0.

In a further aspect the invention relates to a method of treating a subject suffering from an inflammatory process caused by an infection, from an infection or from an infectious process in the *cavitas oris* by applying a physiologically acceptable and pharmacologically active amount of a glycerophosphate derivative according to general formula I or a glycerol phosphate/s according to the invention, a polyglycerol phosphate according to the invention or a lipoteichoic acid according to the invention as described and defined above (also a (general) lipoteichoic acid) to the subject. Preferably the infection is a bacterial infection, the infectious process is bacterial infectious process and/or the inflammation is caused by a bacterial infection, preferably wherein the infection is caused by gram-positive bacteria, the infectious process is caused by gram-positive bacteria and/or the inflammation is caused by gram-positive bacteria.

In a further aspect the invention relates to a method of treating a subject suffering from dental caries or periodontal diseases preferably *caries dentium* by applying a physiologically acceptable and pharmacologically active amount of a glycerophosphate derivative according to general formula I or a glycerol phosphate, a polyglycerol phosphate or a lipoteichoic acid as described and defined above (also a (general) lipoteichoic acid) to the subject.

In a further aspect the invention relates to a method of prophylactically treating a subject in danger of suffering from dental caries by applying a physiologically acceptable and pharmacologically active amount of a glycerophosphate derivative according to general formula I or a glycerol phosphate, a polyglycerol phosphate or a lipoteichoic acid as described and defined above (also a (general) lipoteichoic acid) to the subject.

Preferably all the above methods of treatment or of prophylactically treating involve use of a composition. The composition preferably takes the form of a toothpaste, a toothgel, a cream or a rinsing fluid and/or the glycerophosphate derivative according to general formula I or glycerol phosphate, polyglycerol phosphate or lipoteichoic acid of the invention (also a (general) lipoteichoic acid) is present in an amount between 0.1 to 100 mg/ml, preferably 1 to 10 mg/ml, more preferably 2 to 5 mg/ml, most preferably 3 to 3.5 mg/ml or 0.02 to 2.0 weight-%, preferably 0.05 to 1.0 mg/ml, more preferably 0.1 to 0.75 weight-%, most preferably 0.2 to 0.4 weight-% of the product. It may also be present in an amount between 0.1 to 100 mg/ml, preferably 0.5 to 10 mg/ml, more preferably 0.7 to 7 mg/ml, most preferably 2 to 5 mg/ml or most preferably 3 to 3.5 mg/ml (especially if a lipoteichoic acid) of the composition in the product. Further the composition may further comprise substances selected from:
a fluoride or a sweetener,
preferably (in case of a tooth paste, tooth gel or tooth cream) selected from a fluoride, an abrasive, an enzyme, a surfactant, an oxidizer, a flavouring substance, a sweetener, a binder, a humectant or a preservative, or
preferably (in case of a rinsing fluid) selected from water, an alcohol, a buffering substance, a sweetener, a flavouring substance, a preservative, a fluoride, an enzyme, or an oxidizer.

The composition preferably also shows a pH ≥ 5.5, preferably a pH ≥ 7.0.

Without limitation the invention is further described by way of examples and figures below:

### Figures:

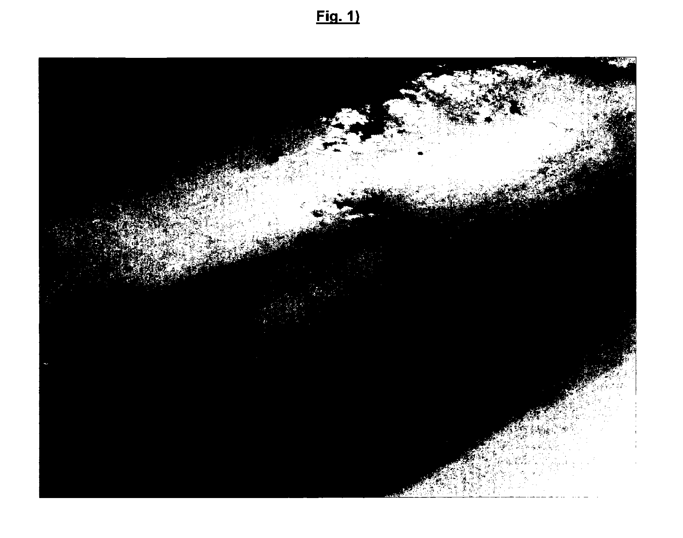

**Fig. 1****:** Picture of the adamantine without *Streptococcus mutans* without treatment (control/PBS).

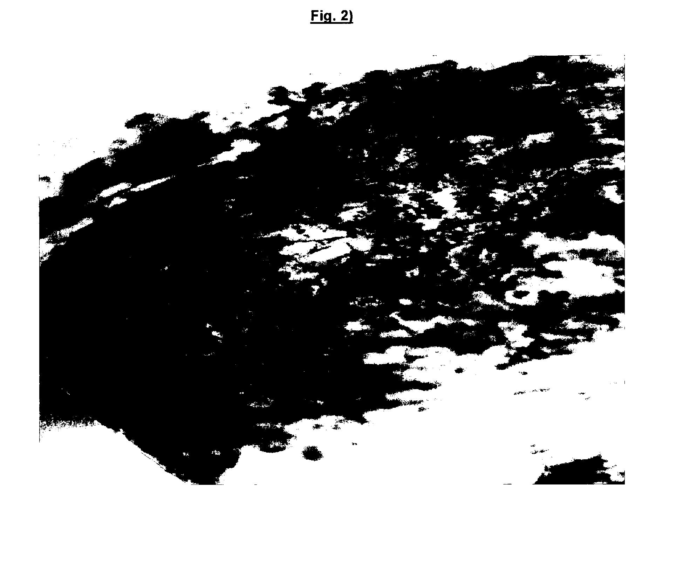

**Fig. 2****:** Picture of the adamantine with *Streptococcus mutans* without treatment (control/PBS).

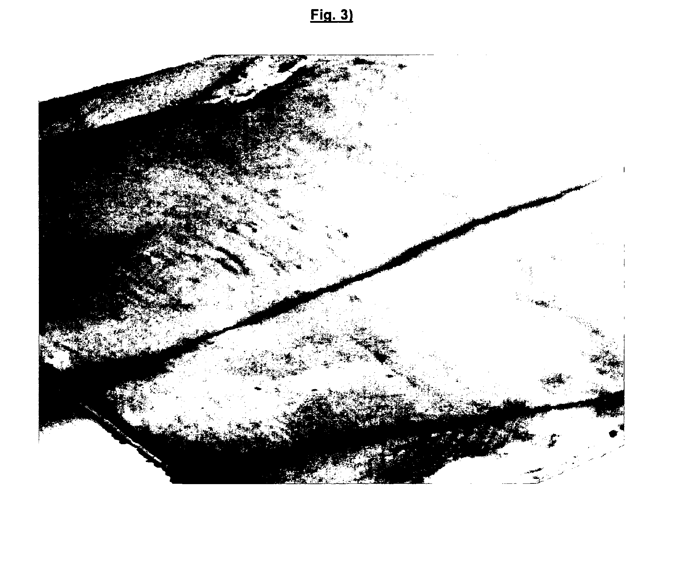

**Fig. 3****:** Picture of the adamantine with *Streptococcus mutans* with LTA-T treatment (see below).

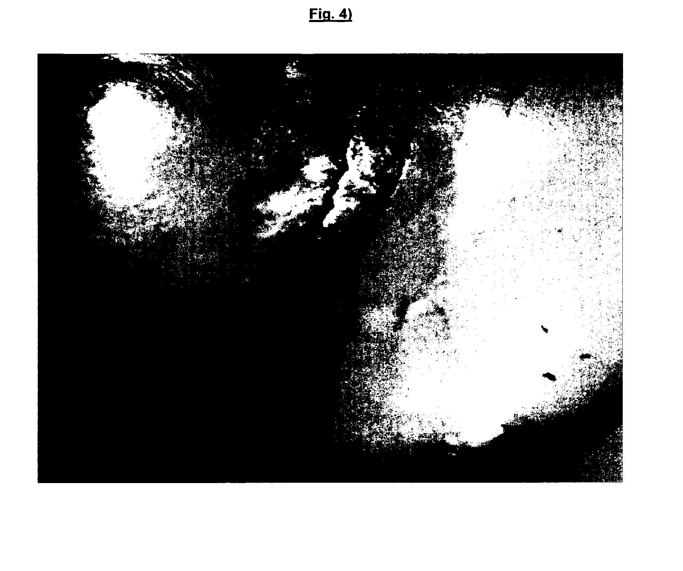

**Fig. 4****:** Picture of the adamantine with *Streptococcus mutans* with glycerol-phoshate treatment (lower dose; see below).

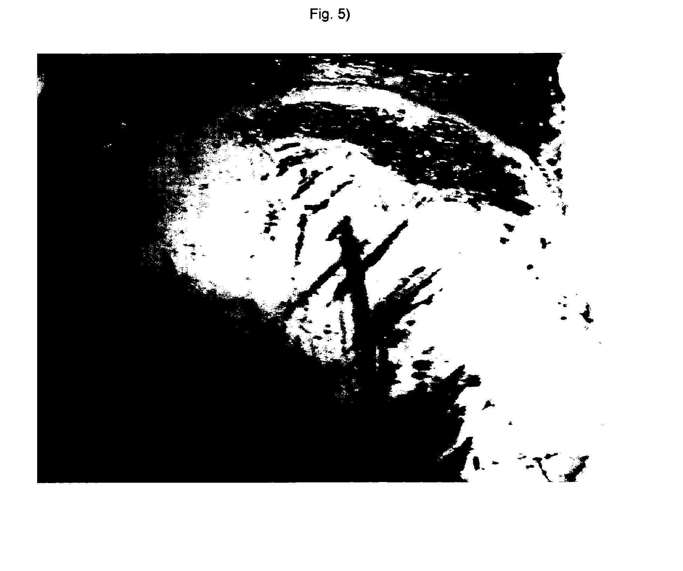

**Fig. 5****:** Picture of the adamantine with *Streptococcus mutans* with polyglycerol-phoshate treatment (lower dose; see below).

### Examples:

### Example 1: Experimental Use in Caries Prophylaxis

Overal 3 different compounds (glycerol phosphate, polyglycerol phosphate (PGP1) and LTA-T) in different concentrations were tested to determine the prophylactic activity against caries.

### 1.1. PROCEDURE

### 1.1.1. Tooth experiment

To investigate the influence of glycerol phosphate, polyglycerol phosphate and LTA-T on the biofilm formation of *Streptococcus mutans* on human teeth, incisors (1/condition) were preincubated under rotating conditions (50 rpm) at 37°C for 1 h with:
1. 5000 µg/ml glycerol phosphate
2. 20000 µg/ml glycerol phosphate
3. 277 µg/ml polyglycerol phosphate (PGP1)
4. 1106 µg/ml polyglycerol phosphate (PGP1)
5. 25 µg/ml LTA-T
6. PBS as control
These teeth were then washed with ultrapure H₂O by repetitive dipping (3 liquid air interfaces) and incubated in a saturated suspension of *Streptococcus mutans* (in BHI broth, 3% sucrose) under rotating conditions (50 rpm) at 37°C for 3 days. Each day, bacteria were fed with 1 ml of a solution of 30% sucrose in PBS to enhance biofilm formation.
After incubation, the teeth were washed with ultrapure H2O, air dried for 2 h and stained with Safranin (0.5 % v/v) solution. The stained teeth were air dried and photographed (see above).

### 1.1.2. Statistical analysis

Of each tooth 4 representative pictures were taken. The pictures were analyzed with SigmaPlot Software, Version 11.0 using phase analysis: The color (phase) of the bacterial biofilm was defined and % area of the chosen color on each picture was calculated.

### 1.2. RESULTS

### 1.2.1 % Area covered with bacteria

| **Treatment** **[Fig.]** | **% Area covered with bacteria** | **% Mean area covered** | **Standard deviation** |
|---|---|---|---|
| Untreated (with bacteria) [Fig. 2] | 56.84 | 37.55 | 13.61 |
| | 29.89 | | |
| | 37.09 | | |
| | 26.38 | | |
| Untreated (without bacteria) [Fig. 1 ] | 1.94 | 0.63 | 0.90 |
| | 0.08 | | |
| | 0.47 | | |
| | 0.00 | | |
| Pre-treatment 5000 µg/ml glycerol phosphate: [Fig. 4] | 0.00 | 0.00 | 0.01 |
| | 0.00 | | |
| | 0.02 | | |
| | 0.00 | | |
| Pre-treatment 20000 µg/ml glycerol phosphate: | 0.00 | 0.00 | 0.00 |
| | 0.00 | | |
| | 0.00 | | |
| | 0.00 | | |
| Pre-treatment 277 µg/ml polyglycerol phosphate [PGP1]: [Fig. 5] | 1.93 | 0.65 | 0.86 |
| | 0.19 | | |
| | 0.08 | | |
| | 0.40 | | |
| Pre-treatment 1106 µg/ml polyglycerol phosphate [PGP1]: | 17.56 | 15.16 | 5.15 |
| | 7.51 | | |
| | 16.97 | | |
| | 18.61 | | |
| Pre-treatment 25 µg/ml LTA-T: [Fig. 3] | 0.00 | 0.03 | 0.05 |
| | 0.00 | | |
| | 0.10 | | |
| | 0.00 | | |

### 1.2.2. Unpaired t-test

The difference between groups was compared using the unpaired t-test.

| **Groups compared** | **Normality test** | **Statistical test used** | **p value** | **significant?** |
|---|---|---|---|---|
| Pre-treatment 5000 µg/ml glycerol phosphate vs. untreated (with bacteria) | Failed (P<0.05) | Mann-Whitney Rank Sum Test | 0.029 | Yes |
| Pre-treatment 2000 µg/ml glycerol phosphate vs. untreated (with bacteria) | Failed (P<0.05) | Mann-Whitney Rank Sum Test | 0.029 | Yes |
| Pre-treatment 277 µg/ml polyglycerol phosphate [PGP1] vs. untreated (with bacteria) | Failed (P<0.05) | Mann-Whitney Rank Sum Test | 0.029 | Yes |
| Pre-treatment 1106 µg/ml polyglycerol phosphate [PGP1] vs. untreated (with bacteria) | Passed (P=0.256) | Unpaired t-Test | 0.022 | Yes |
| Pre-treatment 25 µg/ml LTA-T vs. untreated (with bacteria) | Failed (P<0.05) | Mann-Whitney Rank Sum Test | 0.029 | Yes |

### 1.3. SUMMARY

The teeth with pre-treatment were compared to the control tooth (no pre-treatment, with bacteria). The adhesion of bacteria was significantly reduced after pre-treatment with 5000 µg/ml and 20000 µg/ml glycerol phosphate, 277 µg/ml polyglycerol phosphate and after pre-treatment with 25 µg/ml LTA-T. After pre-treatment with 1106 µg/ml polyglycerol phosphate [PGP1], bacteria were still able to adhere to the tooth surface, but the decrease of biofilm formation was still significant compared to the control tooth.

### Example 2: Toothpaste CO-LTA-T

75 ml of Tooth Paste contains:

| | |
|---|---|
| Sodium fluoride | (1450 ppm F⁻) |
| Lipoteichonic Acid (LTA-T) | 250 µg |

Other Ingredients:
Aqua pura, sorbitol, hydrated silica, glycerin, sodium lauryl sulfate, PEG-12 (polyethylene glycol), cellulose gum, tetrasodium pyrophosphate, cocamidolpropyl betaine, xanthan gum, sodium saccharine, methylparaben, propylparaben, limonene, colorants: CI 74160 = [29H,31H-phthalocyaninato(2-)-N29,N30,N31,N32] copper; CI 74260 (pigment green 7); CI 77891 (titanium dioxide).

### Example 3: Toothpaste CO-PGP

75 ml of Tooth Paste contains:

| | |
|---|---|
| Sodium fluoride | (1450 ppm F⁻) |
| Polyglycerol phosphate of formula IId | |
| wherein n is 3; R¹ is OH; and R² is OH [= PGP1]: | 50 - 500 µg |

Other Ingredients:
Aqua pura, sorbitol, hydrated silica, glycerin, sodium lauryl sulfate, PEG-12 (polyethylene glycol), cellulose gum, tetrasodium pyrophosphate, cocamidolpropyl betaine, xanthan gum, sodium saccharine, methylparaben, propylparaben, limonene, colorants: Cl 74160 = [29H,31H-phthalocyaninato(2-)-N29,N30,N31,N32] copper; CI 74260 (pigment green 7); CI 77891 (titanium dioxide).

### Example 4: Toothpaste CO-SGP

75 ml of Tooth Paste contains:

| | |
|---|---|
| Sodium fluoride | (1450 ppm F⁻) |
| Sodium glycerolphosphate | 50 - 500 µg |

Other Ingredients:
Aqua pura, sorbitol, hydrated silica, glycerin, sodium lauryl sulfate, PEG-12 (polyethylene glycol), cellulose gum, tetrasodium pyrophosphate, cocamidolpropyl betaine, xanthan gum, sodium saccharine, methylparaben, propylparaben, limonene, colorants: CI 74160 = [29H,31H-phthalocyaninato(2-)-N29,N30,N31,N32] copper; CI 74260 (pigment green 7); CI 77891 (titanium dioxide).

### Example 5: Toothpaste OD-LTA-T

75 ml of Tooth Paste contains:

| | |
|---|---|
| Sodium fluoride | (1450 ppm F⁻) |
| Lipoteichonic Acid (LTA-T) | 250 µg |

Other Ingredients:
Aqua pura, sorbitol, hydrated silica, glycerine, sodium lauryl sulfate, PEG-6 (polyethylene glycol), xanthan gum, acrylates/C10-30 Alkyl acrylate crosspolymer, sodium saccharine, chondrus crispus (Carageenan),Sodium hydroxide, limonene, colorants: CI 74160 = [29H,31H-phthalocyaninato(2-)-N29,N30,N31,N32] copper; CI 73360 (6-chloro-2-(6-chloro-4-methyl-3-oxobenzo[b]thien-2(3H)-ylidene)-4-methylbenzo[b]thiophene-3(2H)-one); CI 77891 (titanium dioxide).

### Example 6: Toothpaste OD-PGP

75 ml of Tooth Paste contains:

| | |
|---|---|
| Sodium fluoride | (1450 ppm F⁻) |
| Polyglycerol phosphate of formula IId | |
| wherein n is 3; R¹ is OH; and R² is OH [= PGP1]: | 50 - 500 µg |

Other Ingredients:
Aqua pura, sorbitol, hydrated silica, glycerine, sodium lauryl sulfate, PEG-6 (polyethylene glycol), xanthan gum, acrylates/C10-30 Alkyl acrylate crosspolymer, sodium saccharine, chondrus crispus (Carageenan),Sodium hydroxide, limonene, colorants: CI 74160 = [29H,31H-phthalocyaninato(2-)-N29,N30,N31,N32] copper; Cl 73360 (6-chloro-2-(6-chloro-4-methyl-3-oxobenzo[b]thien-2(3H)-ylidene)-4-methylbenzo[b]thiophene-3(2H)-one); CI 77891 (titanium dioxide).

### Example 7: Toothpaste OD-SGP

75 ml of Tooth Paste contains:

| | |
|---|---|
| Sodium fluoride | (1450 ppm F⁻) |
| Sodium glycerolphosphate | 50 - 500 µg |

Other Ingredients:
Aqua pura, sorbitol, hydrated silica, glycerine, sodium lauryl sulfate, PEG-6 (polyethylene glycol), xanthan gum, acrylates/C10-30 Alkyl acrylate crosspolymer, sodium saccharine, chondrus crispus (Carageenan),Sodium hydroxide, limonene, colorants: CI 74160 = [29H,31H-phthalocyaninato(2-)-N29,N30,N31,N32] copper; CI 73360 (6-chloro-2-(6-chloro-4-methyl-3-oxobenzo[b]thien-2(3H)-ylidene)-4-methylbenzo[b]thiophene-3(2H)-one); CI 77891 (titanium dioxide).

### Example 8: Toothpaste EL-LTA-T

75 ml of Tooth Paste contains:

| | |
|---|---|
| Sodium fluoride (in form of the Aminofluoride Olaflur) | 1440 ppm Fluoride |
| Lipoteichonic Acid (LTA-T) | 250 µg |

Other Ingredients:
Aqua pura, sorbitol, hydrated silica, polyethylene glycol, saccharine, limonene, titanium dioxide.
The pH of this product is between 4.5 and 5.0.

### Example 9: Toothpaste EL-PGP

75 ml of Tooth Paste contains:

| | |
|---|---|
| Sodium fluoride (in form of the Aminofluoride Olaflur) | 1440 ppm Fluoride |
| Polyglycerol phosphate of formula IId | |
| wherein n is 3; R¹ is OH; and R² is OH [= PGP1]: | 50 - 500 µg |

Other Ingredients:
Aqua pura, sorbitol, hydrated silica, polyethylene glycol, saccharine, limonene, titanium dioxide.
The pH of this product is between 4.5 and 5.0.

### Example 10: Toothpaste EL-SGP

75 ml of Tooth Paste contains:

| | |
|---|---|
| Sodium fluoride (in form of the Aminofluoride Olaflur) | 1440 ppm Fluoride |
| Sodium glycerolphosphate | 50 - 500 µg |

Other Ingredients:
Aqua pura, sorbitol, hydrated silica, polyethylene glycol, saccharine, limonene, titanium dioxide.
The pH of this product is between 4.5 and 5.0.

### Example 11: Tooth Jelly EJ-LTA-T

100 g of Tooth Jelly contains:

| | |
|---|---|
| Sodium fluoride | 2.210 g |
| Aminofluoride Dectaflur: | 0.287 g |
| Aminofluoride Olaflur: | 3.032 g |
| overall | (1.25 weight-% fluoride) |
| Lipoteichonic Acid (LTA-T) | 400 µg |

Other Ingredients:
Aqua pura, propylene glycol, hydroxyethyl cellulose, saccharin, flavours.

### Example 12: Tooth Jelly EJ-PGP

100 g of Tooth Jelly contains:

| | |
|---|---|
| Sodium fluoride | 2.210 g |
| Aminofluoride Dectaflur: | 0.287 g |
| Aminofluoride Olaflur: | 3.032 g |
| overall | (1.25 weight-% fluoride) |
| Polyglycerol phosphate of formula IId | |
| wherein n is 3; R¹ is OH; and R² is OH [PGP1]: | 50 - 800 µg |

Other Ingredients:
Aqua pura, propylene glycol, hydroxyethyl cellulose, saccharin, flavours.

### Example 13: Tooth Jelly EJ-SGP

100 g of Tooth Jelly contains:

| | |
|---|---|
| Sodium fluoride | 2.210 g |
| Aminofluoride Dectaflur: | 0.287 g |
| Aminofluoride Olaflur: | 3.032 g |
| overall | (1.25 weight-% fluoride) |
| Sodium glycerolphosphate | 50 - 800 µg |

Other Ingredients:
Aqua pura, propylene glycol, hydroxyethyl cellulose, saccharin, flavours.

## Claims

1. Glycerophosphate derivative according to general formula I; 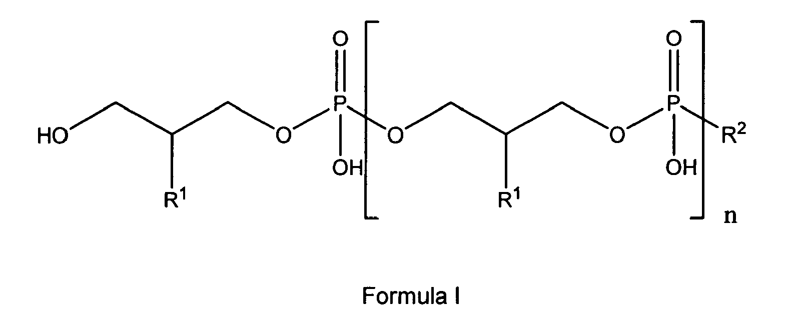 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
either 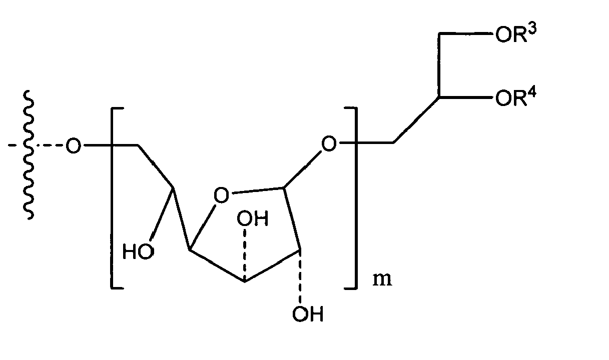 or its tautomeric equivalent 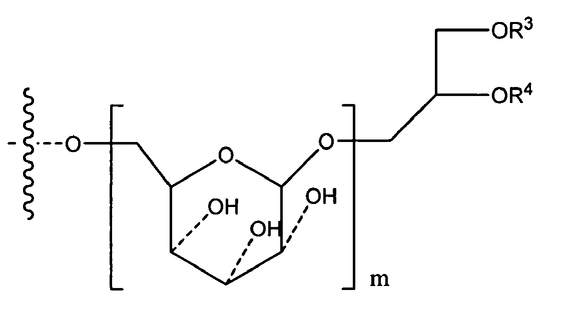 with R³ and R⁴ independently from one another being residues of saturated or unsaturated, unsubstituted fatty acids with 10 to 20 carbon atoms; and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures;
for use in the treatment of or prophylaxis against an inflammatory process caused by an infection, of or against an infection or of or against an infectious process in the *cavitas oris.*

2. Glycerophosphate derivative according to claim 1, wherein the compound of formula I is a glycerol phosphate according to any of the following formulas V, Va, Vb or Vc: 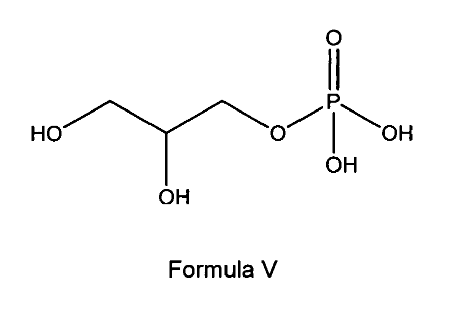 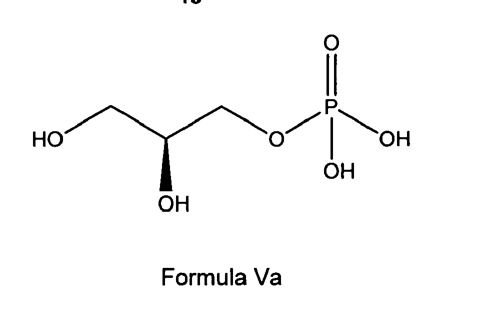 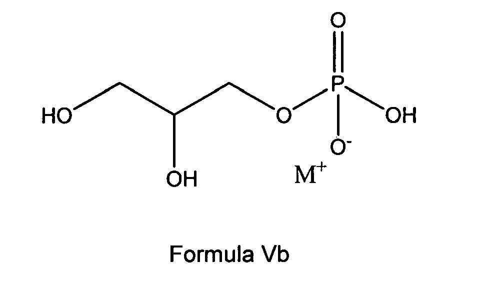 or 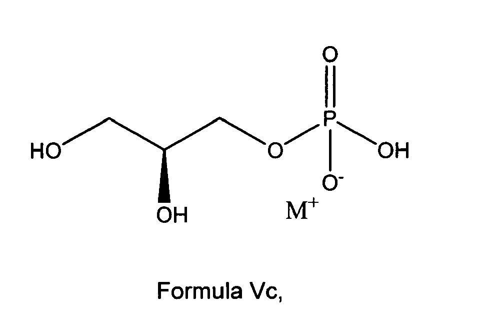 wherein M⁺ is selected from positively charged ions, such as alkali metal ions or positively charged primary, secondary, tertiary or quaternary ammonium ions, or M⁺ is a sodium ion.

3. Glycerophosphate derivative according to claim 1, wherein the compound of formula I is selected from free glycerol phosphate, sodium glycerol phosphate, potassium glycerol phosphate or magnesium glycerol phosphate, preferably is sodium glycerolphosphate (or free glycerol phosphate).

4. Glycerophosphate derivative according to claim 1 wherein the glycerophosphate derivative is a polyglycerol phosphate according to general formula I; 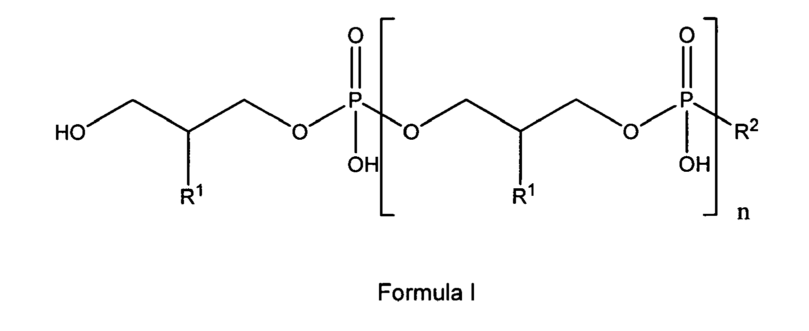 wherein
n is 1 to 11,
R¹ is either H, OH or OMe or OEt and
R² is OH or -O-CH₂-CH(R¹)-CH₂-OH;
with the compounds of formula I being present either in free form or as physiologically acceptable salt; in form of any of their structural isomers including mixtures thereof, preferably in pure enantiomeric or diastereomeric form or any mixture thereof including racemic mixtures.

5. Glycerophosphate derivative according to claim 4 wherein the polyglycerol phosphate is a compound according to any of general formulas II, IIa, IIb, IIc, IId or IIe 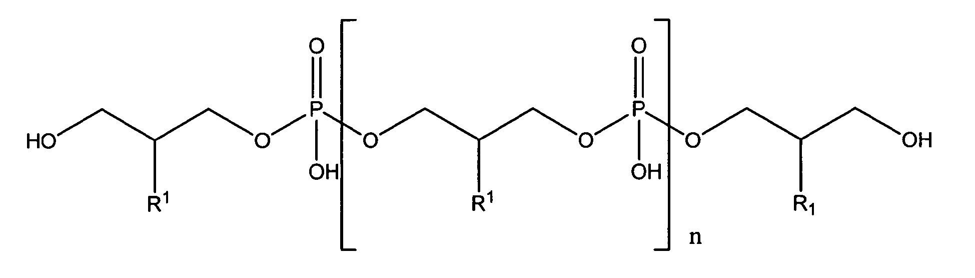 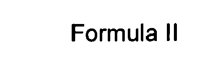 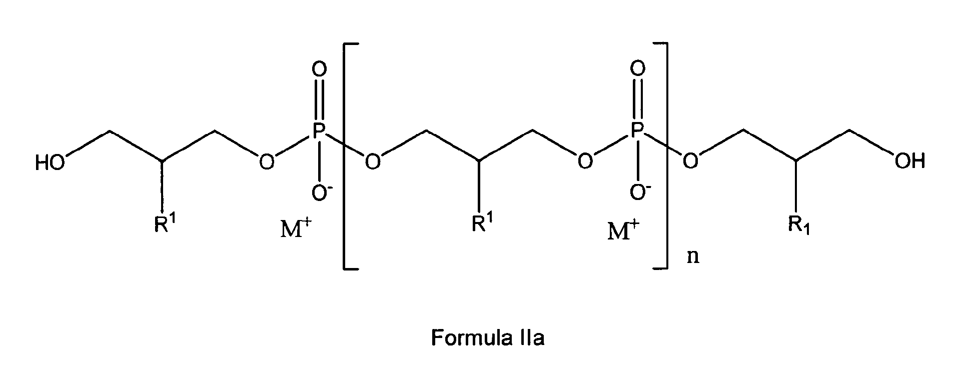 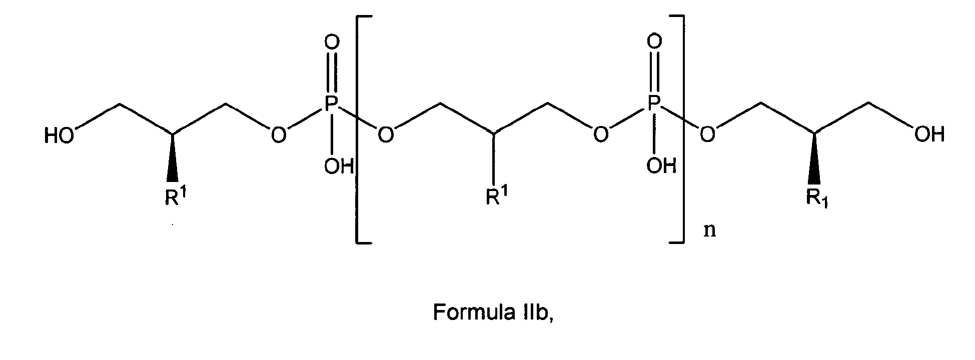 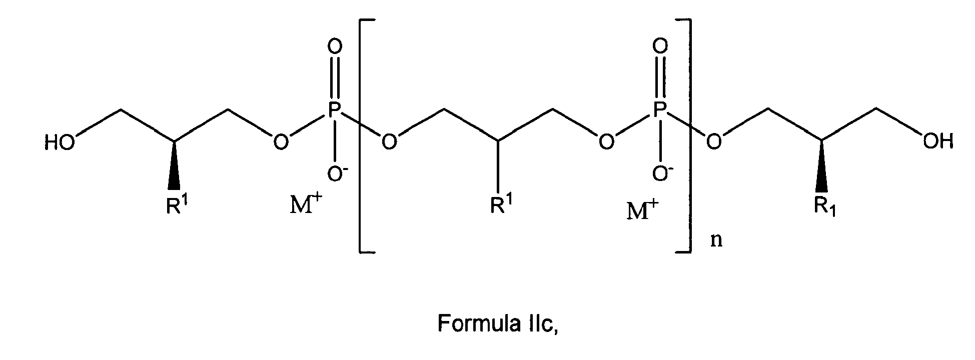 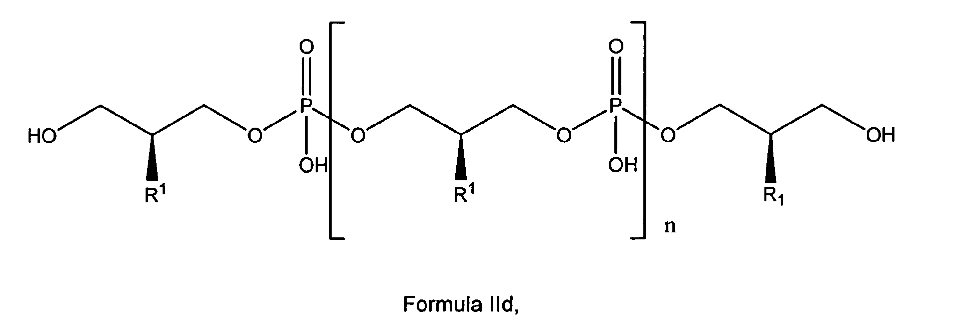 or 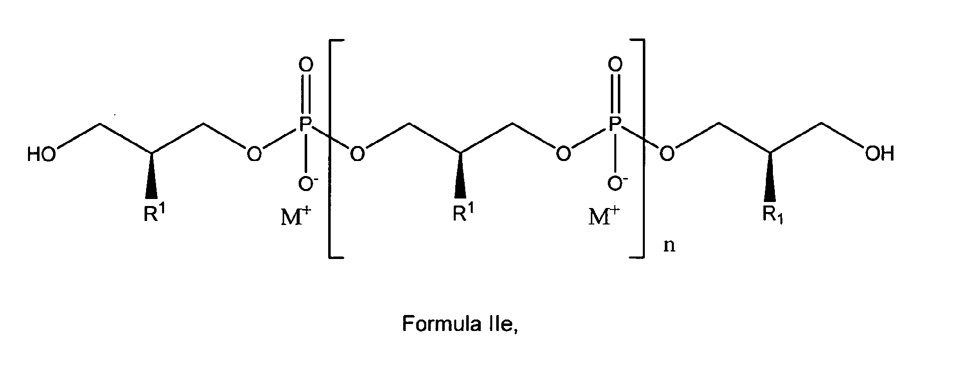 wherein
n is 0 to 10, or n is 1 to 10;
R¹ is either H, OH or OMe or OEt, or R¹ is OH; and
M⁺ is selected from positively charged ions, such as alkali metal ions or positively charged primary, secondary, tertiary or quaternary ammonium ions, or M⁺ is a sodium ion.
most preferably wherein the polyglycerol phosphate is a compound according to general formula IId 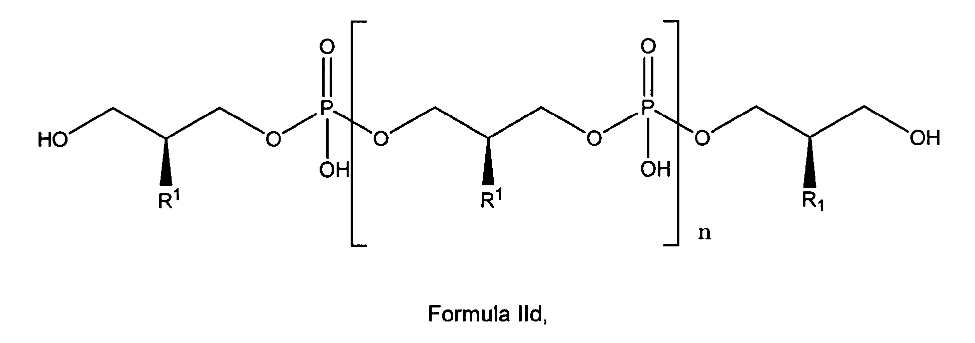 wherein
n is 3; and
R¹ is OH;
either in free form or as physiologically acceptable salt.

6. Glycerophosphate derivative according to claim 1 wherein the glycerophosphate derivative is a lipoteichoic acid according to general formula I 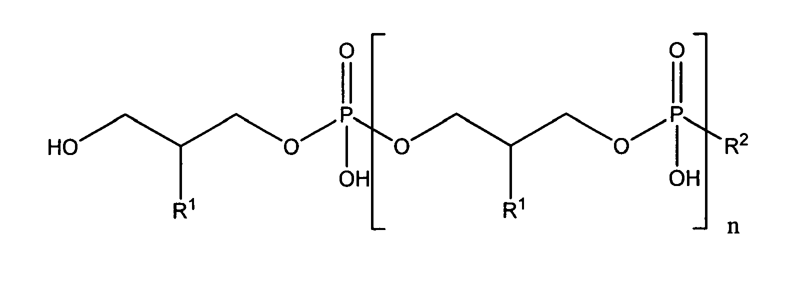 wherein
the mean value of n is 8 to 40,
R¹ is either OH or O-D-Alanyl and
R² is
either 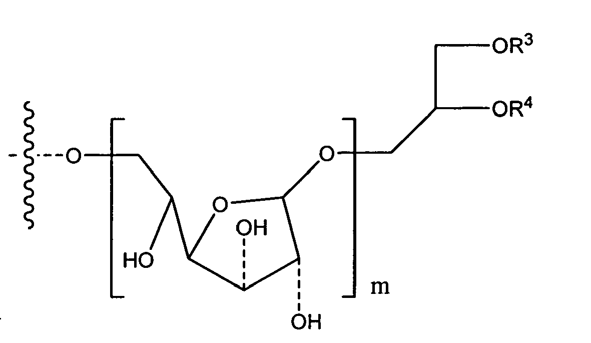 or its tautomeric equivalent 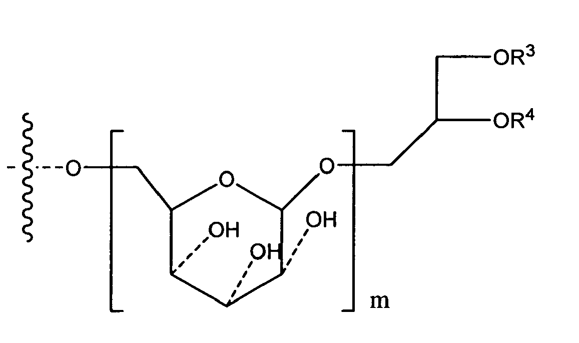 with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

7. Glycerophosphate derivative according to claim 6 wherein the glycerophosphate derivative is a lipoteichoic acid according to general formula I 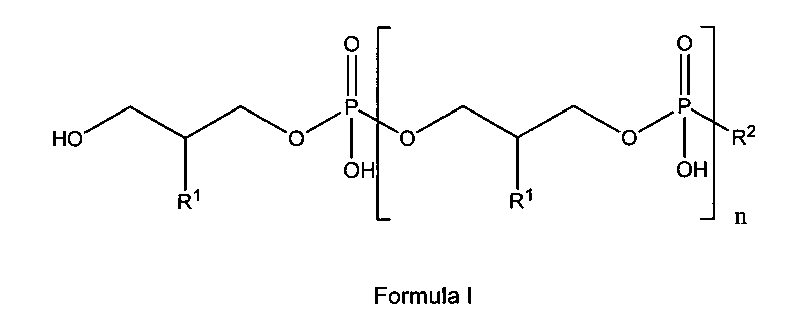 wherein
the mean value of n is 9,
R¹ is either OH or O-D-Alanyl and
R² is 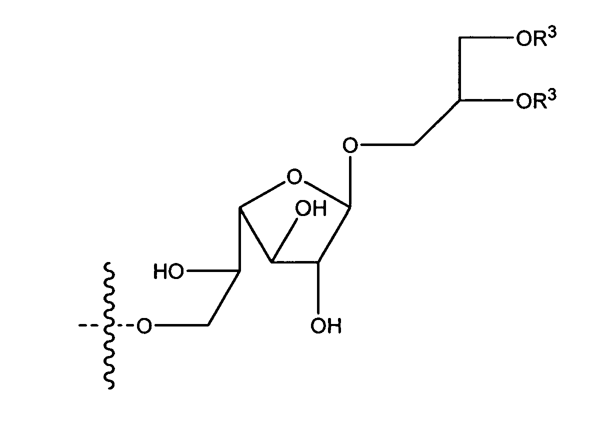 with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures.

8. Glycerophosphate derivative according to claim 7 wherein the lipoteichoic acid is a compound according to any of general formulas III, IIIa, IIIb, or IIIc 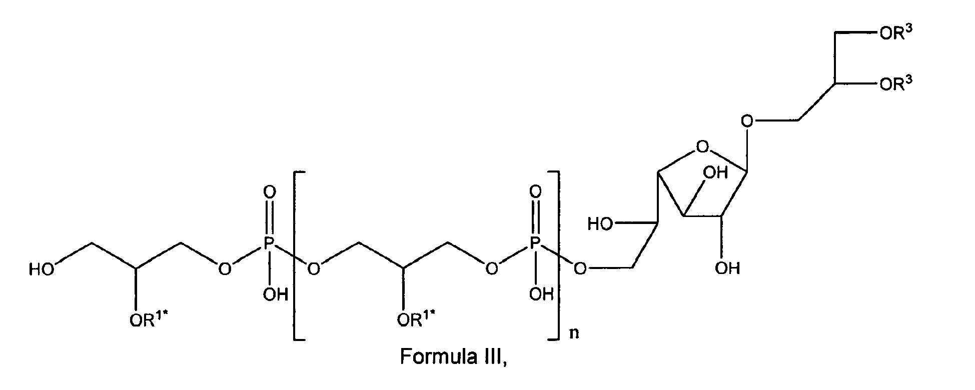 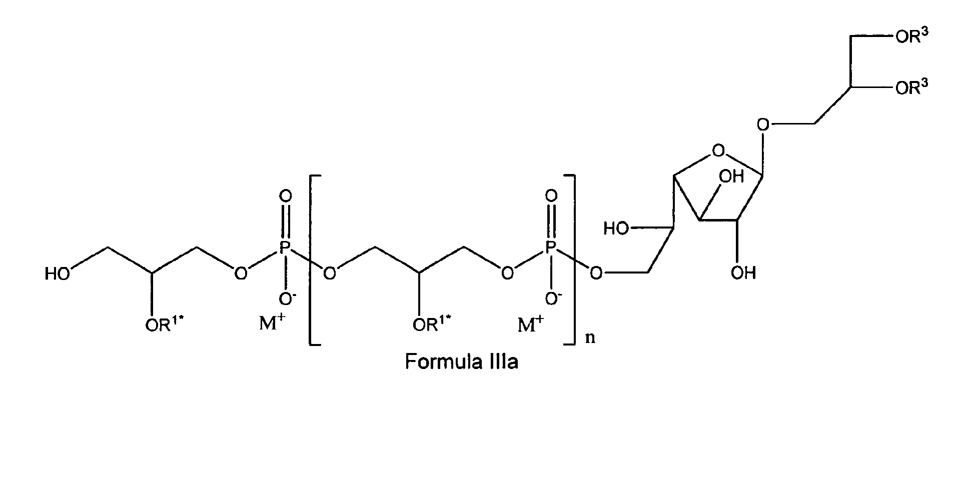 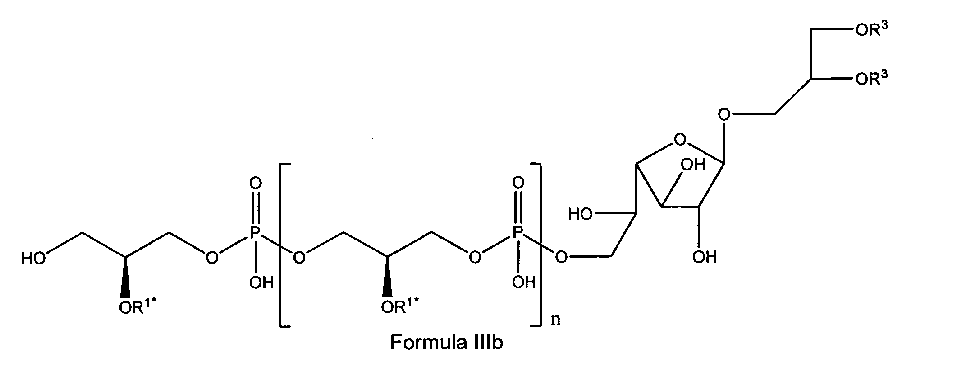 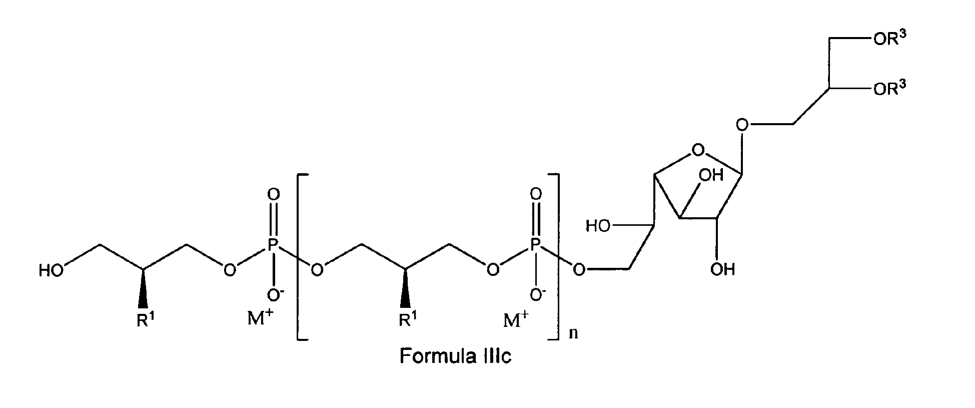 with
n having a mean value of 9, preferably being 6 to 12 while having a mean value of 9, R¹* being either H or D-Alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

9. Glycerophosphate derivative according to claims 6 to 8 wherein the lipoteichoic acid is a purified lipoteichoic acid (LTA-T) isolated from bacteria of the genus *streptococcus,* preferably from *streptococcus sp.,* most preferably from *streptococcus sp.* (DSM 8747).

10. Glycerophosphate derivative according to any of claims 1 to 9, wherein the infection is a bacterial infection, the infectious process is bacterial infectious process and/or the inflammation is caused by a bacterial infection, preferably wherein the infection is caused by gram-positive bacteria, the infectious process is caused by gram-positive bacteria and/or the inflammation is caused by gram-positive bacteria;
and/or
wherein the infectious process is *caries dentium* or periodontal diseases preferably is *caries dentium* or wherein the use is in the treatment of or prophylaxis against *caries dentium* or periodontal diseases preferably of or against *caries dentium.*

11. Glycerophosphate derivative according to any of claims 1 to 10, wherein the use includes use of an oral or dental hygiene product, preferably the use wherein the glycerophosphate derivative is used in form of a toothpaste, a toothgel, a cream or a rinsing fluid.

12. Glycerophosphate derivative according to claim 11, wherein the glycerophosphate derivative according to general formula I is present in the composition in the oral or dental hygiene product in an amount between 0.1 to 100 mg/ml, preferably 0.5 to 10 mg/ml, more preferably 0.7 to 7 mg/ml, most preferably 2 to 5 mg/ml.

13. Glycerophosphate derivative according to claim 11 or 12, wherein the composition in the oral or dental hygiene product further comprises substances selected from
a fluoride or a sweetener,
preferably (in case of a tooth paste, tooth gel or tooth cream) selected from a fluoride, an abrasive, an enzyme, a surfactant, an oxidizer, a flavouring substance, a sweetener, a binder, a humectant or a preservative, or
preferably (in case of a rinsing fluid) selected from water, an alcohol, a buffering substance, a sweetener, a flavouring substance, a preservative, a fluoride, an enzyme, or an oxidizer.

14. Glycerophosphate derivative according to claim 11 to 13, wherein the composition in the oral or dental hygiene product shows a pH ≥ 5.5, preferably a pH ≥ 7.0.

15. A dental hygiene product comprising a glycerophosphate derivative according to general formula I; 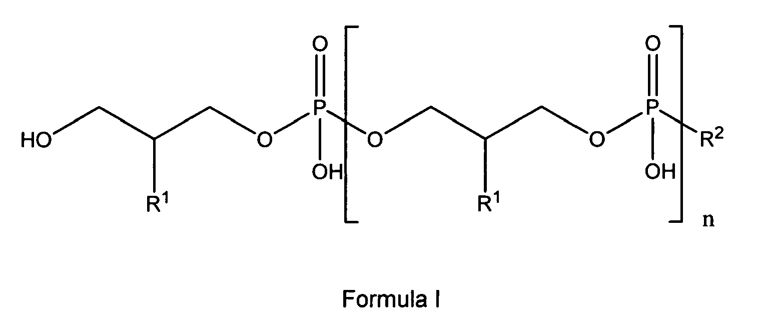 wherein
n is either 0, 1 to 11 or has a mean value of 9;
and
if n is 0, R¹ and R² are OH;
if n is 1 to 11, R¹ is either H, OH or OMe or OEt and R² is OH or -O-CH₂-CH(R¹)-CH₂-OH;
if n has a mean value of 8 to 40, R¹ is either OH or O-D-Alanyl and R² is
either 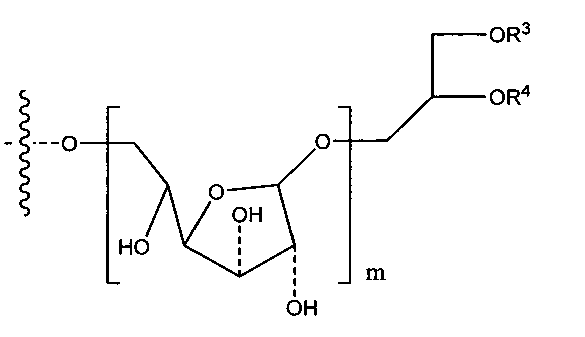 or its tautomeric equivalent 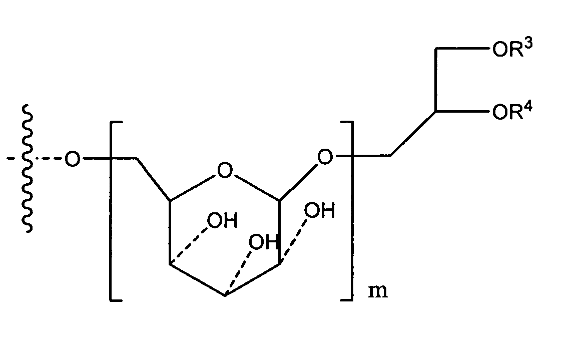 with R³ and R⁴ independently from one another being residues of saturated or unsaturated, unsubstituted fatty acids with 10 to 20 carbon atoms; and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.
